# EUROPÄISCHE PATENTSCHRIFT

(11) **EP 3 158 335 B1**
(45) Veröffentlichungstag und Bekanntmachung des Hinweises auf die Patenterteilung: **14.08.2019**
(21) Anmeldenummer: 15731541.7
(22) Anmeldetag: 17.06.2015
(51) Int. Cl.: G01N 33/564, C07K 14/72, G01N 33/543

(54) **NACHWEIS VON AUTOANTIKÖRPERN GEGEN DEN TSH-REZEPTOR**
DETECTION OF ANTI-TSH RECEPTOR AUTOANTIBODIES
DÉTECTION D'AUTO-ANTICORPS ANTIRÉCEPTEURS DE LA TSH

(30) Priorität: 20.06.2014 EP 14173341; 11.08.2014 DE 102014011653
(43) Veröffentlichungstag der Anmeldung: 26.04.2017
(73) Patentinhaber: Loos, Ulrich, 89081 Ulm (DE)
(72) Erfinder: Loos, Ulrich, 89081 Ulm (DE)
(74) Vertreter: Cohausz & Florack
(86) Internationale Anmeldenummer: PCT/EP2015/063627
(87) Internationale Veröffentlichungsnummer: WO 2015/193387

(56) Entgegenhaltungen:
- WO-A1-2007/036511
- TAHARA K ET AL: "EPITOPES FOR THYROID STIMULATING AND BLOCKING AUTOANTIBODIES ON THEEXTRACELLULAR DOMAIN OF THE HUMAN THYROTROPIN RECEPTOR", THYROID, MARY ANN LIEBERT, NEW YORK, NY, US, Bd. 7, Nr. 6, 1. Dezember 1997 (1997-12-01), Seiten 867-877, XP000993366, ISSN: 1050-7256

## Beschreibung

### TECHNISCHES GEBIET DER ERFINDUNG

Die Erfindung betrifft ein auf einem Diagnose-Automaten durchführbares Verfahren nach Anspruch 1 zum Nachweis von stimulierenden Autoantikörpern bzw. Autoimmunantikörpern gegen den Schilddrüsenhormonrezeptor.

### TECHNOLOGISCHER HINTERGRUND DER ERFINDUNG

Der TSH-Rezeptor (TSH-R) spielt eine Schlüsselrolle für Wachstum und Funktion von Schilddrüsenzellen. Dieser Rezeptor ist ein Glied einer Unterfamilie der G-Protein gekoppelten Glycoproteinrezeptoren, die außerdem die Rezeptoren für das luteinisierende Hormon/Choriongonadotropin (LH/CGR) und das Follikel stimulierende Hormon (FSHR) umfasst. Die Rezeptoren dieser Unterfamilie weisen eine große N-terminale extrazelluläre Domäne auf, die für die Ligandenbindung von essentieller Bedeutung ist und für die gezeigt wurde, dass sie an der Signalübermittlung beteiligt ist. Die Übermittlung des TSHR-Signals wird überwiegend durch Aktivierung von A-denylatzyklase vermittelt, was zu einer Erhöhung des intrazellulären cAMP-Niveaus führt.

Ein Teil des großen Interesses an dem TSH-Rezeptor ist auf dessen Rolle als primäres Autoantigen bei Schilddrüsen-Autoimmunkrankheiten zurückzuführen, die vom Auftreten von Autoantikörpern gegen den TSH-Rezeptor begleitet sind. Zu derartigen Schilddrüsen-Autoimmunerkrankungen gehört die Basedowsche Krankheit, eine zu Schilddrüsenüberfunktion führende Autoimmunerkrankung, die zu den häufigsten menschlichen Autoimmunerkrankungen überhaupt gehört. Die Basedowsche Krankheit wird durch Aktivierung der Adenylatcyclase und resultierenden cAMP-Anstieg verursacht. Dadurch kommt es zur Schilddrüsen-Überfunktion, Kropfbildung und gegebenenfalls Augenveränderungen. Die Autoantikörper gegen den TSH-Rezeptor können aber auch blockierenden Charakter haben und somit die Adenylatcyclase und das cAMP hemmen. In diesem Falle kommt es zu einer Unterfunktion der Schilddrüse. Ein gleichzeitiges Auftreten von stimulierenden und blockierenden Autoantikörpern bei den betroffenen Patienten ist ebenfalls möglich, wobei der Anteil der stimulierenden Autoantikörper in der Regel überwiegt.

Zum Nachweis solcher Autoantikörper gibt es seit einiger Zeit einen Bioassay, in dem die cAMP-Zunahme gemessen wird. Dieses Messverfahren ist sehr zeitaufwendig. Darüber hinaus ist der Bioassay nicht zuverlässig, da er falsch positive Ergebnisse liefern kann. Im Rahmen dieser Beschreibung wird diese Art von Messverfahren als Bioassay zur Abgrenzung von den in-vitro-Verfahren zur Bestimmung von Autoantikörpern gegen den TSH-Rezeptor bezeichnet. Bei einem auf dem Markt befindlichen in-vitro-Nachweisverfahren für Autoantikörper wird ein aus Schweineschilddrüsenmembran extrahierter TSH-Rezeptor eingesetzt (erste Generation der in-vitro-Verfahren). In einem anderen Test zum Nachweis von Autoantikörpern gegen den TSH-Rezeptor wird ein humanes vollständiges rekombinantes TSH-Rezeptorprotein (Wildtyp) in einem Kompetionsstest eingesetzt.

Thyroid Vol.7 (1997) 867-877 beschreibt die Epitope für stimulierende und blockierende Autoantikörper am TSH-Rezeptor. Die Mehrzahl der funktionellen Epitope für stimulierende Autoantikörper sind im Bereich der Aminosäuren 8 bis 168 und diejenigen für die blockierenden Autoantikörper im Bereich der Aminosäuren 261 bis 370 des Rezeptorproteins vorhanden. Für Aktivitätsmessungen wird der zuvor genannte Bioassay eingesetzt.

Durch die WO 01/27634 A1 wird erstmalig ein quantitatives Verfahren zum Nachweis von Autoantikörpern unterschiedlicher Spezifität nebeneinander bereitgestellt, das schnell reproduzierbar und mit hoher Genauigkeit durchführbar ist. Zu diesem Zweck werden TSH-Rezeptorchimären eingesetzt, die sich von dem Wildtyp-Rezeptor dadurch unterscheiden, dass einzelne Sequenzen, an denen Autoantikörper binden, ausgetauscht sind durch entsprechende Sequenzen eines anderen Rezeptors aus der Klasse der G-Protein gekoppelten Rezeptoren. Die TSH-Rezeptorchimären umfassen ansonsten den vollständigen TSH-Rezeptor. Allerdings ist der Messaufwand hoch. Das Trennen durch Zentrifugieren macht das Verfahren zu umständlich für eine routinemäßige Nutzung. Auch muss der Test im Eisbad oder bei 4°C durchgeführt werden, da die TSH-Rezeptorchimären nicht sehr stabil sind. Eine entsprechende technische Lehre findet sich in der WO 01/63296 A1, in der die Verwendung einer Sandwich-Technik zur Detektion vorgeschlagen wird. Es hat sich jedoch herausgestellt, dass in der Regel mit den dort vorgeschlagenen Testmaterialien, die unspezifische Bindung zu hoch ist. Ein Test zum Nachweis von Autoantikörpern auf der Grundlage der Erkenntnisse der beiden zuvor genannten Patentanmeldungen ist auf dem Markt nicht erhältlich.

Die WO 00/00590 A1 beschreibt einen C-terminal trunkierten TSH-Rezptor und in allgemeiner Weise dessen Eignung zur Diagnose und Therapie. Die WO 2007/036511 A1 beschreibt einen sogenannten Bridge-Essay zum Nachweis von Autoantikörpern gegen den TSH-Rezeptor, der erstmalig auf einem Automaten durchführbar ist. Zur Durchführung dieses Bridge-Assays werden zwei TSH-Rezeptorchimären eingesetzt, wobei die signalgebende Rezeptorchimäre nur den extrazellulären Bereich des TSH-Rezeptors umfasst und die sogenannte Anker-Rezeptorchimäre ein Holoenzym ist. Dieses Verfahren wird ab Frühjahr 2014 weltweit zur automativen Diagnose von gegen den Schilddrüsenhormonrezeptor gerichteten Autoantikörpern angeboten werden und wird auf dem sogenannten Immulite®-Automaten durchgeführt. Der Ankerrezeptor ist an Kunststoffteilchen eines Durchmessers von beispielsweise etwa 6 mm als Festphase gebunden.

### ZUSAMMENFASSUNG DER ERFINDUNG

Wünschenswert ist die Anwendung des Verfahrens der WO 2007/036511 A1 auch auf vollautomatischen Analyseeinrichtungen, die mit paramagnetischen Teilchen als Festphase arbeiten. Weder ein Test mit den TSH-Rezeptorchimären der WO 2007/036511 A1 noch ein Test mit zwei TSH-Rezeptoren, die nur die extrazelluläre Domäne des TSH-Rezeptors umfassen, ermöglicht die Durchführung des Nachweisverfahrens auf einem Vollautomaten, der mit paramagnetischen Teilchen arbeitet wie dem ADVIA Centaur-Automaten oder dem ARCHITECT-Automaten.

Die Verwendung von zwei extrazellulären TSH-Rezeptorchimären (trunkierte TSH-Rezeptorchimären) als Anker- und als Signalrezeptor zeigte eine für die Durchführung des in der WO 2007/036511 A1 beschriebenen Bridge-Assays zu geringe Sensitivität und Spezifität zum Nachweis von Autoantikörpern gegen den TSH-Rezeptor. Eine mit dieser TSH-Rezeptorchimären-konstellation durchgeführte Messung erwies sich als nicht reproduzierbar, was in Beispiel 3 (Vergleichsbeispiel) gezeigt ist.

Es ist aber äußerst erstrebenswert, ein Verfahren zum Nachweis von Autoantikörpern gegen den Schilddrüsenhormonrezeptor bereitzustellen, das genau dieselbe Genauigkeit und Aussagekraft wie das in der WO 2007/036511 A1 beschriebene Verfahren hat und darüber hinaus auf einem Vollautomaten unter Einsatz von paramagnetischen Teilchen als Festphase möglich ist.

Gelöst wird diese Aufgabe durch ein auf einem Diagnose-Automaten durchführbares Verfahren zum Nachweis von stimulierenden Autoantikörpern gegen den TSH-Rezeptor gemäß Anspruch 1.

Überraschend können nun erstmalig zwei trunkierte TSH-Rezeptorchimären in einem Bridge-Assay zum Nachweis von Autoantikörpern gegen den TSH-Rezeptor eingesetzt werden. Von der vollständigen TSH-Rezeptorchimäre wird also erstmalig in einem Brückenassay nur die extrazelluläre Domäne der Rezeptorchimäre eingesetzt. Überraschend wurde ferner festgestellt, dass zum Nachweis von Autoantikörpern gegen den TSH-Rezeptor als Ankerrezeptor für einen Autoantikörper eine an paramagnetischen Teilchen immobilisierte gebundenen erste TSH-Rezeptorchimäre aus einem den extrazellulären Teil des TSH-Rezeptors umfassenden Protein und als Signalrezeptor eine zweite TSH-Rezeptorchimäre aus einem den extrazellulären Teil des TSH-Rezeptors umfassenden Protein, geeignet ist, wenn der extrazelluläre Teil des Rezeptors mit einem zur Sezernierung des TSH-Rezeptors in den Extrazellulärraum geeigneten Protein fusioniert ist. Die erfindungsgemäß eingesetzten TSH-Rezeptorchimären besitzen eine hohe Lagerstabilität, Transportstabilität, Reservoirstabilität und auch eine hohe Assaystabilität.

### BESCHREIBUNG DER BEVORZUGTEN AUSFÜHRUNGSFORMEN

Der TSH-Rezeptor erstreckt sich vom Cytosol durch die Zellmembran in den Extrazellulärraum. Der extrazelluläre Anteil des Rezeptorproteins weist die Bindungsstellen für TSH und die Autoantikörper auf.

Ist die Anzahl der Aminosäuren der einfindungsgemäß eingesetzten TSH-Rezeptorchimäre zu hoch, verschlechtert sich bei deren Gewinnung die Sezernierung der TSH-Rezeptorchimäre aus den zur Erzeugung eingesetzten rekombinanten Zellen in den Extrazellulärraum.

Die erste und gegebenenfalls die zweite TSH-Rezeptorchimäre können zusätzlich eine Aminosäuresequenz des cytosolischen Teils des Rezeptors enthalten. Hierzu kommen vorzugsweise die Aminosäuren 698-725 des cytosolischen Teils des TSH-Rezeptors oder Teilsequenzen davon in Betracht. Dabei handelt es sich um eine hochimmunogene Aminosäuresequenz, die sich als vorteilhaft zur Immobilisierung der ersten TSH-Rezeptorchimäre an den paramagnetischen Teilchen erwiesen hat.

Als wesentlich für die Durchführung des erfindungsgemäßen Verfahrens hat sich erwiesen, dass der extrazelluläre Teil der Rezeptorchimären mit einem die Sezernierung der Rezeptorchimäre verursachenden Protein fusioniert ist. Ein solches Protein ist beispielsweise ein Serumprotein eines Säugers. Vorzugsweise wird Albumin verwendet. Die Fusionierung erfolgt N-terminal. Die erste erfindungsgemäß eingesetzte TSH-Rezeptorchimäre enthält ein Serumprotein. Vorzugsweise wird Albumin verwendet, dass nach Fusion mit der extrazellulären Domäne des TSH-Rezeptors die TSH-Rezeptorchimäre stabilisiert und zur Sezernierung der erfindungsgemäß eingesetzten TSH-Rezeptorchimäre aus der Zelle, in der die Rezeptorchimäre erzeugt wird, führt. Dasselbe gilt für ein Fusionsprotein aus der extrazellulären Domäne des Wildtyp-TSH-Rezeptors und einem Serumprotein. Die erste eingesetzte TSH-Rezeptorchimäre kann auch nur Teilsequenzen eines Serumproteins, insbesondere Albumin-Proteins, umfassen soweit diese die Stabilisierung der TSH-Rezeptorchimäre und die Sezernierung aus der Zelle gewährleisten. Durch diese Maßnahme wird der TSH-Rezeptorchimäre Stabilität verliehen.

Die zweite erfindungsgemäß eingesetzte TSH-Rezeptorchimäre weist grundsätzlich denselben Aufbau auf wie die erste erfindungsgemäß eingesetzte TSH-Rezeptorchimäre. Da die zweite TSH-Rezeptorchimäre im erfindungsgemäßen Verfahren die Funktion eines Signalgebers aufweist, muss sie sich insoweit von der ersten TSH-Rezeptorchimäre (Ankerrezeptor) unterscheiden. Zu diesem Zweck weist sie ein Markierungsmittel auf. Das Markierungsmittel kann N-terminal oder C-terminal an der zweiten TSH-Rezeptorchimäre fusioniert sein. Vorzugsweise ist das Markierungsmittel C-terminal an der zweiten TSH-Rezeptorchimäre fusioniert. Gemäß einer bevorzugten Ausführungsform der Erfindung weist die Signal-TSH-Rezeptorchimäre N-terminal eine sekretorische alkalische Phosphatase auf. Die sekretorische alkalische Phosphatase kann auch zu einer Detektionsreaktion verwendet werden. Auf eine weitere Modifizierung am C-terminalen Teil der zweiten TSH-Rezeptorchimäre zur Detektion kann dann verzichtet werden. Es sei denn, die Detektion soll durch eine andere Reaktion erfolgen.

Sollen stimulierende Autoantikörper nachgewiesen werden, wird man eine Rezeptorchimäre einsetzen, bei der die Sequenz ausgetauscht ist, die blockierende oder neutrale Autoantikörper gegen den TSH-Rezeptor bindet.

Zum Austausch werden vorzugsweise vergleichbare Sequenzen eines anderen Rezeptors verwendet, die im Wesentlichen keine Bindung für die jeweils beiden anderen Typen von Autoantikörpern aufweisen. Solche vergleichbaren Sequenzen können beispielsweise Sequenzen eines Ratten LG-CG-Rezeptors sein. Bei den erfindungsgemäß eingesetzten TSH-Rezeptorchimären können folglich die Epitope, an denen stimulierende, neutrale und/oder blockierende Autoantikörper binden, ausgetauscht sein. Diese Rezeptorchimären können gemäß Biochem. Biophys. Res. Comun. (1991), 179:70-77 oder WO 01/27634 A2 konstruiert werden. Auf beide Dokumente wird im Hinblick auf die Erzeugung von TSH-Rezeptorchimären Bezug genommen.

Im Falle des Nachweises von stimulierenden Autoantikörpern sind die Bindungssequenzen für blockierende und neutrale Antikörper im TSH-Rezeptorprotein ausgetauscht durch keine Bindung des jeweilig anderen Typs von Autoantikörpern bewirkende Sequenzen eines anderen Rezeptors. Bei der zum Nachweis stimulierender Autoantikörper verwendeten Chimäre können vorzugsweise die TSH-Rezeptoraminosäuren 261 bis 370 durch im Wesentlichen die entsprechenden Aminosäuren 261 bis 329 eines Ratten-LH-CGR ersetzt sein.

Besonders vorteilhaft an den erfindungsgemäß eingesetzten TSH-Rezeptorchimären ist, dass sie mit einer Aminosäuresequenz zur Sezernierung der TSH-Rezeptorchimäre aus der Zelle fusioniert sind und so bei deren Produktion in rekombinanten Zellen, in den Extrazellulärraum sezerniert werden. Ein Aufschluss von Zellen zur Gewinnung der TSH-Rezeptorchimären ist dann nicht mehr erforderlich. Damit lässt sich die erfindungsgemäß zu verwendende TSH-Rezeptorchimäre mit einem hohen Reinheitsgrad und im Wesentlichen ohne Kontaminierung mit Zellbestandteilen gewinnen, die im Falle eines Zellaufschlusses zur Gewinnung des Proteins anfallen.

Die erfindungsgemäß eingesetzte zweite TSH-Rezeptorchimäre (Signalrezeptor) kann zur Detektion modifiziert sein, beispielsweise kann sie je nach gewünschtem Assaydesign mit einem Markierungsmittel fusioniert sein. Die Markierungen können derart sein, dass sie unmittelbar oder mittelbar ein nachweisbares Signal liefern. Eine direkte Markierung im Sinne der Erfindung ist eine Markierung der TSH-Rezeptorchimäre mit einem unmittelbar ein nachweisbares Signal zur Detektion des Immunkomplexes liefernden Markierungsmittel. Eine indirekte Markierung im Sinne der Erfindung kann beispielsweise die Fusion der TSH-Rezeptorchimäre mit einer immunogenen Peptidsequenz sein, die durch einen zur Detektion geeigneten sekundären markierten Antikörper erkannt wird.

Markierungsmittel können durch Fusion oder chemische Bindung mit der TSH-Rezeptorchimäre oder den sekundären Antikörpern verbunden sein. Geeignete Markierungen erfolgen beispielsweise durch Enzyme wie alkalische Phosphatase (AP), sekretorische alkalische Phosphatase (SEAP), Leuchtkäfer- oder Gausialuciferase und Peroxidase oder einen Farbstoff wie einen Acridinfarbstoff, ein fluoreszierendes oder bio-/chemieluminiszierendes Material. Im Falle der zuvor genannten Enzyme ist die dafür codierende Nukleotidsequenz vorzugsweise mit der Nukleotidsequenz der zweiten TSH-Rezeptorchimäre (Signalrezeptor) fusioniert. Geeignete Markierungen erfolgen beispielsweise ferner durch Fluoresceinisothiocyanat (FITC), Biotinylierung und Streptavidin. In einer bevorzugten Ausführungsform kann die zweite TSH-Rezeptorchimäre N-terminal mit sekretorischer alkalischer Phosphatase zur Detektion fusioniert sein.

In einer weiteren bevorzugten Ausführungsform ist die zweite TSH-Rezeptorchimäre C-terminal mit einem Acridinfarbstoff direkt markiert.

In dem erfindungsgemäßen Verfahren ist die sogenannte erste TSH-Rezeptorchimäre während der Immunkomplexbildung an einer paramagnetischen festen Phase gebunden. Die Bindung der TSH-Rezeptorchimäre an dieser festen Phase kann über einen immobilisierenden Antikörper erfolgen, der beispielsweise gegen ein C-terminales Epitop der TSH-Rezeptorchimäre gerichtet ist. Ein solcher Antikörper kann ein polyklonaler Antikörper oder ein monoklonaler Antikörper sein. Beispielsweise kann der immobilisierende Antikörper gegen die Aminosäuren 698-725 des cytosolischen Teils der ersten TSH-Rezeptorchimäre gerichtet sein.

Zum Nachweis des Bindens eines Autoantikörpers an der TSH-Rezeptorchimäre kann auch ein sekundärer Antikörper eingesetzt werden. Der sekundäre Antikörper kann zusätzlich zu dem immobilisierenden Antikörper vorhanden sein. Er kann ein monoklonaler oder ein polyklonaler Antikörper sein.

Geeignete feste Phasen umfassen paramagnetische Teilchen, wie paramagnetische Röhrchen, paramagnetische Plättchen und paramagnetische Partikel, aber auch andere Morphologien paramagnetischer Teilchen. Solche paramagnetischen Teilchen sind dem Fachmann bekannt und im Handel beispielsweise unter der Marke Dynabeads® der Firma Invitrogen Dynal AS erhältlich. Die erfindungsgemäß als feste Phase eingesetzten paramagnetischen Teilchen können einen Durchmesser von beispielsweise 0,5 µm bis 5 µm aufweisen. Die Teilchen können paramagnetisch oder superparamagnetisch sein.

Die erfindungsgemäße eingesetzte TSH-Rezeptorchimäre kann lyophilisiert gelagert werden. Sie können über einen monoklonalen oder polyklonalen Antikörper an eine Festphase gebunden in lyophilisierter Form gelagert werden. Für die Nachweisreaktion erfolgt eine Rekonstitution der lyophilisierten Komponenten durch Lösen in einem Assaypuffer.

Die erfindungsgemäße eingesetzte TSH-Rezeptorchimäre besitzt in gelöster Form eine hohe Stabilität (Assay- und Reservoirstabilität). Sie bleiben bei 4°C sechs bis sieben Tage lang stabil und können nach erneuter Kalibration des Diagnose-Automaten weitere zwei bis drei Wochen lang eingesetzt werden. Bei einer Temperatur von 37°C bleiben die gelösten erfindungsgemäßen TSH-Rezeptorchimären sechs Stunden lang stabil. Diese Bedingungen eignen sich für die Durchführung des erfindungsgemäßen Nachweisverfahrens auf Diagnose-Automaten, auf denen die Komponenten bei 4°C gelagert werden, während die Versuchsreaktion problemlos bei 37°C ablaufen kann und damit insbesondere auch dem Automaten Advia Centaur. Demgegenüber bleibt die Holo-TSH-Rezeptorchimäre bei 4°C bis zu sechs Tage, bei 24°C bis 48 Stunden und bei 37°C nur drei Stunden lang stabil.

Zur Durchführung des erfindungsgemäßen Verfahrens nach Anspruch 1 wird jeweils eine Patientenprobe mit einer ersten immobilisierten TSH-Rezeptorchimäre (Ankerrezeptor) und einer zweiten TSH-Rezeptorchimäre (Signalrezeptor) umgesetzt und auf den entsprechenden Autoantikörper untersucht.

Beispielhafte Ausführungsformen des erfindungsgemäßen Verfahrens werden nachfolgend unter Bezugnahme auf die Abbildungen beschrieben.
Abbildungen 1 und 2 zeigen schematisch an paramagnetischen Teilchen immobilisierte Immunkomplexe als Produkt des Verfahrens.
Abbildung 3. insbesondere 3.1 und 3.2, zeigt schematisch eine im erfindungsgemäßen Verfahren einsetzbare erste TSH-Rezeptorchimäre (Ankerrezeptor). Dabei handelt es sich um ein Konstrukt von Teilsequenzen hTSHR mit teilweisem Sequenzaustausch gegen Ratten-LH/CG-Rezeptor fusioniert mit Sequenzen des humanen Albumin sowie einem hTSHR-Ankerepitop. Es werden die Schnittstellen angegeben. Die Nummerierungen der Aminosäuren beziehen sich auf die jeweilige Aminosäure bzw. Base des Holorezeptors.
Abbildung 4 zeigt die Ergebnisse des Vergleichsbeispiels. Die Ergebnisse sind im Vergleichsbeispiel erläutert.
Abbildung 5 zeigt die Assaystabilität des Ankerrezeptors, der mit einem Ankerantikörper auf paramagnetischen Teilchen immobilisiert ist, bei Inkubation bei 37 °C und bei 4 °C ohne Schütteln. Nach 3 Stunden bzw. 3,75 Stunden sind noch etwa 80 % oder mehr des Ankerrezeptors intakt.

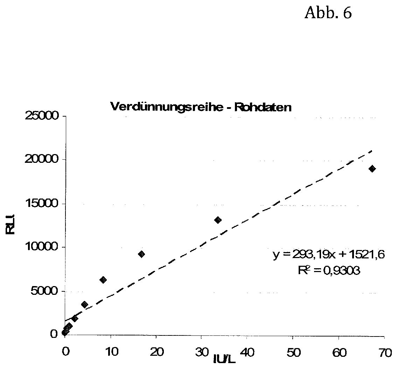

Abbildung 6 zeigt die Linearität einer Verdünnungsreihe eines Patientenserums mit 66,5IU/L. Die Messungen erfolgten durch das erfindungsgemäße Verfahren. Auf der Y-Achse ist die relative Lichteinheit RLU und auf der X-Achse die Konzentration von gemessenen Autoantikörpern als internationale Einheiten pro Liter angegeben.
Abbildung 7 zeigt Messungen zu einer Standardreihe, die kalibriert worden ist mit dem sTRAB-DERA-Brückenassay der EP 1 929 311 B1. Die Kalibrierung erfolgte mit Werten im Mikrotiterplattentest. Das Ergebnis zeigt, dass mit dem erfindungsgemäßen Brückenassay ebenfalls eine hohe Linearität erreicht wird.

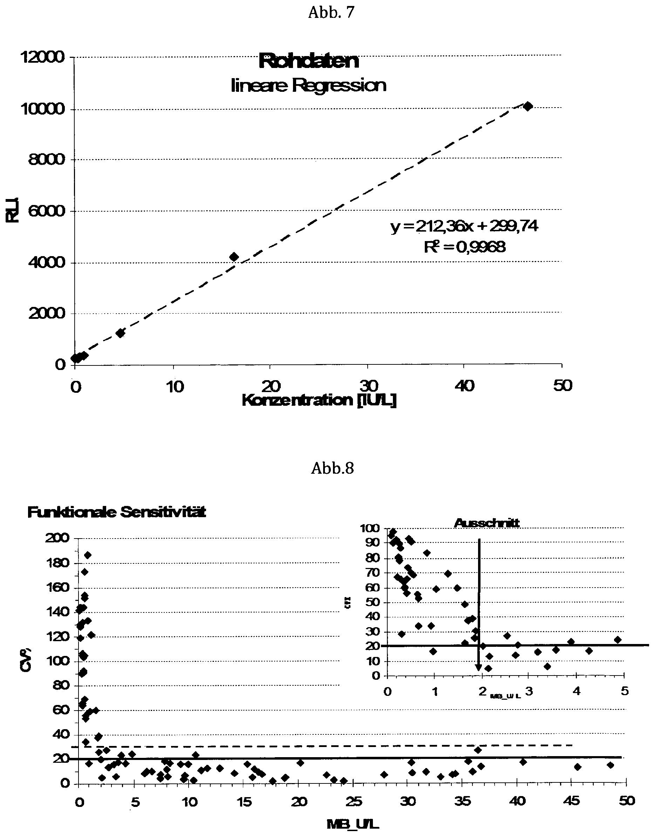

Abbildung 8 dient der Festlegung der funktionalen Sensitivität des erfindungsgemäßen Verfahrens. Die funktionale Sensitivität entspricht hier der 20%-Grenze der Interassay-Varianz. Basierend auf der Bestimmung von 114 Seren und 17 Serenpools (Mehrfachbestimmungen, n = 3 - 7), liegt die vorläufige funktionale Sensitivität bei etwa_1,9 MB (Magnet Beads)_U/L. Unter Berücksichtigung erhöhter Schwankung durch die manuelle Durchführung des Assays kann die funktionale Sensitivität alternativ mit einer Grenze der Interassay-Varianz von 30% ausgewertet werden; in diesem Fall liegt der Wert bei etwa 1,7 MB_U/L. Das erfindungsgemäße Verfahren hat somit eine sehr gute Sensitivität.

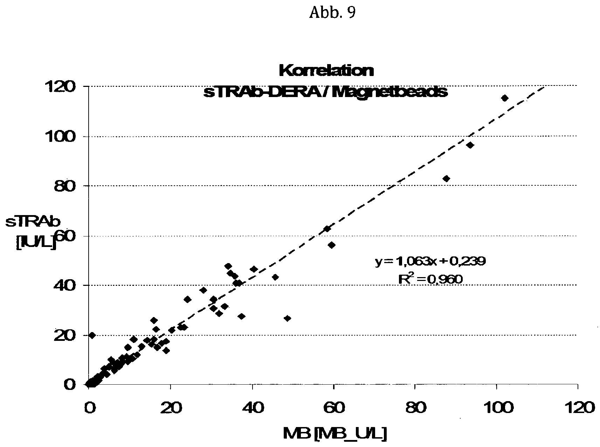

Abbildung 9 zeigt eine gute Korrelation zwischen Ergebnissen mit Magnetbeads-Assay (MB-Werte) und sTRAb-Werten (463 Einzelwerte von 114 Seren und 17 Serenpools).
   SEQ ID Nr.1 zeigt die Nukleotidsequenz eines Ankerrezeptors, mit dem extrazellulären Teil einer TSH-Rezeptorchimäre und einer cytosolischen Aminosäuresequenz einer TSH-Rezeptor-chimäre N-terminal fusioniert mit humanem Serumalbumin einschließlich nicht translatierter Sequenzen.
   SEQ ID Nr.2 zeigt die Aminosäuresequenz zu SEQ ID Nr. 1. In SEQ ID Nr. 1 und 2 (erste TSH-Rezeptorchimäre bzw. Ankerrezeptor) stehen die Nukleotide 1 bis 618 (Aminosäuren 1-206) für die HSA-Peptidsequenz, Nukleotide 619 bis 1553 (Aminosäuren 207-516) für die TSHR-Sequenz der Aminosäuren 21 bis 330 der Chimäre, Nukleotide 1345 bis 1553 (Aminosäuren 446-516) für die LHR-Sequenz der Aminosäuren 261 bis 330 der Chimäre, Nukleotide 1554 bis 1637 (Aminosäuren 516-543) für ein hochimmunogenes Epitop aus dem cytosolischen Teil des TSH-Rezeptors (codiert durch die Nukleotide 743-763 des TSH-Rezeptors).
   SEQ ID Nr. 3 zeigt eine Teilsequenz des extrazellulären Teils des TSH-Rezeptors. In SEQ ID Nr. 5 stehen die Aminosäuren 1 bis 240 für die Aminosäuren 21 bis 260 des extrazellulären Teils des TSH-Rezeptors (ECD260).
   SEQ ID Nr. 4 zeigt eine weitere Teilsequenz des extrazellulären Teils der TSH-Rezeptorchimäre. In SEQ ID Nr. 4 stehen die Aminosäuren 1 bis 310 für die Aminosäuren 21 bis 330 des extrazellulären Teils des TSH-Rezeptors wobei die Aminosäuren 241 bis 310 durch die entsprechenden Aminosäuren 261 bis 330 eines Ratten-LH-CGR ersetzt sind (ECD330).
   SEQ ID Nr. 5 zeigt eine Nukleotidsequenz eines Fusionsproteins aus der extrazellulären Domäne eines Wildtyps des TSH-Rezeptors und einer cytosolischen Aminosäuresequenz N-terminal fusioniert mit humanem Serumalbumin einschließlich nicht translatierter Sequenzen.
   SEQ ID Nr. 6 zeigt die Aminosäuresequenz eines Fusionsproteins aus dem extrazellulären Teil eines Wildtyps des TSH-Rezeptors und einer cytosolischen Aminosäuresequenz N-terminal fusioniert mit humanem Serumalbumin einschließlich nicht translatierter Sequenzen.

Nachfolgend wird die Durchführung des erfindungsgemäßen in vitro-Assay zur direkten Bestimmung von stimulierenden TSH-R-Autoantikörpern auf paramagnetischen Mikrobeads als Matrix auf einer Mikrotiterplatte beschrieben.

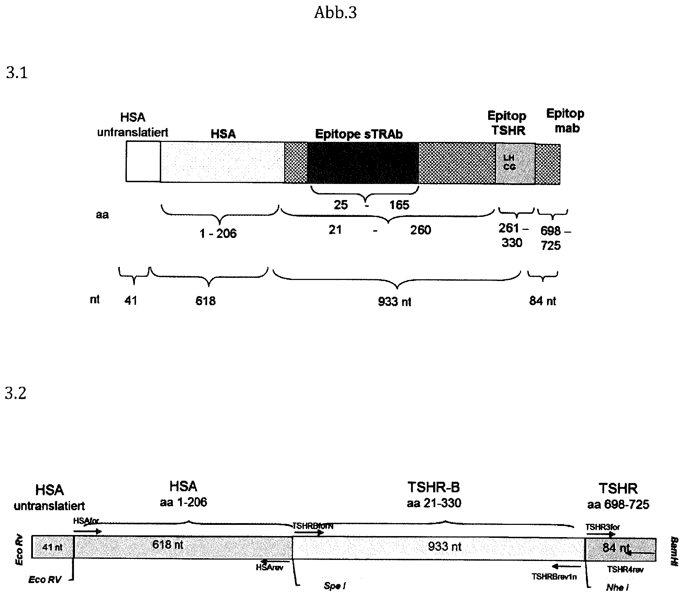

Als Ankerrezeptor auf den paramagnetischen Microbeads als fester Phase ist ein Konstrukt gentechnisch zur Produktion von einem Fusionsprotein wie in Abbildung 3 gezeigt, hergestellt, welches zentral eine TSHR-ECD (extrazelluläre Domäne) mit den Aminosäuren (AA) 21-330 enthält. Diese liegt als Chimäre vor, in welcher die AA 261-330 durch eine korrespondierende LH/CG-R-Sequenz ersetzt ist, damit keine blockierenden Antikörper mitgemessen werden. C-terminal ist die cytosolische TSHR-Sequenz von AA 698-725 fusioniert, an welche zur Verankerung auf den paramagnetischen Microbeads ein monoklonaler Antikörper bindet. N-terminal ist die Teilsequenz von humanem Serumalbumin (AA 1-206) fusioniert. Diese HSA-ECD330-Chim dient als sekretorisches Protein der Freisetzung von dem gesamten Fusionsprotein aus den Kulturzellen in das Medium. Dadurch ist eine einfachere Produktion des Ankerrezeptors für die Bindung von Autoantikörpern möglich. Diese sekretierte Rezeptorchimäre unterscheidet sich durch seinen hohen Reinheitsgrad von der kompletten Wildtyp-Chimäre, welche nach Extraktion aus den Zellen durch Kontamination mit Zellbestandteilen verunreinigt ist. Neben dem Vorteil der Sekretion konnte erstmalig gezeigt werden, dass dieses Fusionsprotein, wenn es an paramagnetische Microbeads gekoppelt wird, eine deutlich höhere Stabilität in Puffer und auch in dem Gemisch aus Puffer und Patientenserum aufweist. Das Humanserumalbumin (HSA) hat erstaunlicherweise eine besondere Bedeutung, denn das Kopplungsprodukt, bestehend aus paramagnetischen Microbeads und daran über Antikörper gebundenem TSHR-Konstrukt, bleibt auf den Automaten bei 4°C über mehrere Tage in gelöster Form stabil (funktional). Das sind die notwendigen Voraussetzungen für einen Assay der paramagnetische Microbeads als feste Phase benutzt. Der komplette chimärische Wildtyp-Rezeptor, der als Extrakt vorliegt, verliert unter diesen gelösten Bedingungen sehr schnell seine Funktionalität und ist daher gekoppelt an paramagnetische Microbeads in Lösung auf Automaten nicht geeignet. Erstaunlicherweise ist das HSA für die Stabilität des Fusionskonstrukts HSA-ECD330-Chim verantwortlich.

In einem zweiten Assayschritt wird Patientenserum mit dem beschriebenen Kopplungsprodukt HSA-ECD330-Chim in Pufferlösung inkubiert. Dann werden die paramagnetischen Microbeads an einen Magnet gebunden, damit anschließend durch Waschung die Empfindlichkeit der Messungen beeinflussende Faktoren eliminiert werden.

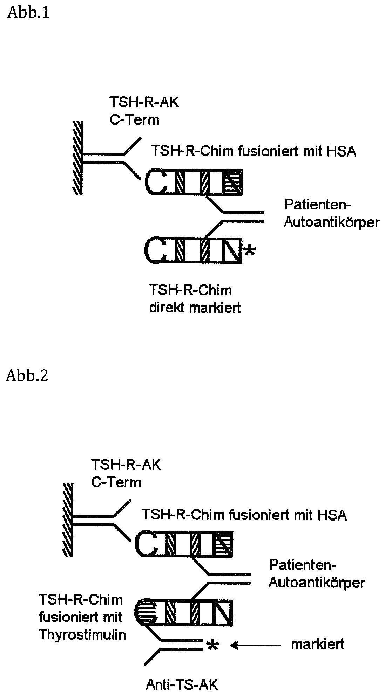

Im dritten Assayschritt wird in das Reaktionsgefäß der Detektionsrezeptor hinzugegeben, der ebenfalls rekombinant als Fusionsprotein vorliegt. Er enthält zentral die ECD als Chimäre, bei der die Aminosäuren 261-266 ebenfalls durch korrespondierende LH/CG-Sequenzen ausgetauscht sind. Der Detektionsrezeptor ist N-terminal mit SEAP (AA 1-520) fusioniert. Der gebildete Immunkomplex kann dann aussehen wie schematisch in den Abbildungen 1 und 2 gezeigt. Ein Magnet wird immer dann aktiviert, wenn Flüssigkeiten (Probe, Reagenzien, Waschpuffer) aus dem Reaktionsgefäß entfernt werden sollen. Die Quantifizierung kann durch Chemolumineszenz über SE-AP-Aktivierung erfolgen; alternativ kann der Detektionsrezeptor mit Acridiniumestern markiert werden.

Wie beschrieben kann die Bestimmung von stimulierenden Autoantikörpern auch derart erfolgen, dass eine Messung durchgeführt wird, in der als TSH-Rezeptoren zur Bindung der stimulierenden Autoantikörper ein TSH-Rezeptorprotein mit der extrazellulären Domäne des Wildtyprezeptors anstelle der TSH-Rezeptorchimären eingesetzt wird. Von dem dabei ermittelten Messwert kann der Wert subtrahiert werden, der bei der Messung mit den TSH-Rezeptorchimären für stimulierende Autoantikörper ermittelt wurde. Auf diese Weise werden auch nicht stimulierende Autoantikörper, beispielsweise blockierende und neutrale Autoantikörper, erfasst. Das kann für die klinische Diagnose von Relevant sein.

Wird das erfindungsgemäße Verfahren zur Analyse von Seren mit blockierenden Autoantikörpern von Patienten, die nachweislich eine behandlungsbedürftige schwere Hypothyreose haben, verwendet, sind diese mit der erfindungsgemäßen Verfahren nicht nachweisbar. Auch das spricht für die Spezifität des erfindungsgemäßen Verfahrens.

Die Erfindung wird durch die folgenden Beispiele weiter erläutert.

### Beispiel 1 (Gewinnung der TSH-Rezeptorchimären)

### Materialien

Für die Klonierung der TSH-Rezeptorchimären wurde das Plasmid pcDNA3-rLHR (B9) von Dr. D.L. Segaloff (The University of Iowa, USA) verwendet.

Die TSH-Rezeptorchimäre, bei der eine Teilsequenz durch eine entsprechende Sequenz eines Ratten-LH-CGR ersetzt ist, wurde gemäß der Beschreibung in Biochem Biophys Res Commun. (1991), 179: 70-77 konstruiert. Dabei waren die TSHR-Aminosäuren 261 bis 370 durch die vergleichbaren Aminosäuren 261 bis 329 eines Ratten LH-CGR ersetzt.

Die TSH-Rezeptorchimären und deren Wildtyp-Variante wurde gemäß gängigen Klonierungsverfahren mit gängigen Reagenzien hergestellt.

### Expression und Gewinnung von Fusionsproteinen HSA2-TSHR-ECD-B-330-mAb1 oder TSHR/LH-CGR-SEAP als Zellextrakt

Konfluente stabile CHO-K1- bzw. HEK293-Zellen werden in Platten gezüchtet. Der Überstand (etwa 8 mg/ml Gesamtprotein) wurde gesammelt und bei -70 °C aufbewahrt. Der so erhaltene Überstand kann in Bestimmungsverfahren als HSA2-TSHR-ECD-B-330-mAb1, TSHR-SEAP bzw. TSHR/LH-CGR-SEAP eingesetzt werden.

### Gewinnung der sekretierten extrazellulären Fusions-TSHR-Chimären

Die trunkierten, extrazellulären Domänen der Fusions-TSH-R-Chimären werden von den sie exprimierenden Zellen in den Kulturüberstand sekretiert. Die sekretierten Rezeptor-Proteine werden in Form von bestimmten Verdünnungen im Assaypuffer direkt in den Versuch eingesetzt. Zum Beispiel werden 10 µl extrazelluläre SEAP-TSH-R-Chimäre B aus 5 ml Kulturüberstand in einer Endverdünnung von 1:10 pro Bestimmung benutzt.

### Kopplung des Ankerantikörpers an paramagnetische Teilchen

Es werden 10 mg (330 µL) tosylierte Dynabeads® M-280 (Invitrogen Dynal AS) entnommen, zweimal mit jeweils 1 mL 0,1 M Boratpuffer pH 9,5 gewaschen und anschließend in 330 µL Boratpuffer resuspendiert. Hierzu wird bei jedem Waschschritt folgendermaßen verfahren: Der Magnet wird aktiviert und die Lösung von den paramagnetischen Teilchen entfernt. Dann wird der Magnet deaktiviert und 1 mL 0,1 M Boratpuffer pH 9,5 zu den paramagnetischen Teilchen gegeben und für einige Sekunden gut geschüttelt. Darauf folgen die Aktivierung des Magneten und das Entfernen der Waschlösung von den paramagnetischen Teilchen. Es erfolgt die Zugabe von 4,3 µg Antikörper (0,43 µg Antikörper/mg Dynabeads®). Hierzu wird anti-TSHR Klon 3D7 entnommen und mit Boratpuffer auf 30 µL verdünnt. Des Weiteren werden 30 µL pro 100 µL Dynabeads® einer 3 M, mit 0,1 M Boratpuffer pH 9,5 angesetzten Ammoniumsulfatlösung zugegeben und gründlich gemischt. Es folgt eine Inkubation für mehr als 20 h (über Nacht) bei Raumtemperatur und unter Schütteln im Über-Kopf-Schüttler. Anschließend wird zweimal mit je 1 mL Boratpuffer wie oben beschrieben gewaschen und 0,5 mL frisch hergestellter Blockierungspuffer (Boratpuffer, 0,5 % BSA, 0,05% Tween 20) zugegeben. Die Dispersion wird unter Schütteln für 2 h bei 37°C inkubiert. Im Anschluss werden die Dynabeads® zweimal mit 1 mL frisch hergestelltem Waschpuffer (PBS pH 7,4, 0,1% BSA, 0,1% Tween 20) wie oben beschrieben gewaschen. Dann wird der Überstand abgenommen und die mit Ankerantikörper gekoppelten Dynabeads® in 500 µL Waschpuffer in einer Konzentration von 20 mg/mL resuspendiert.

### Kopplung der ersten TSH-Rezeptorchimäre (Ankerrezeptor) auf paramagnetische Teilchen

30 µL einer Lösung enthaltend 20 mg/mL mit Ankerantikörper gekoppelte Dynabeads® in Puffer (PBS pH 7,4, 0,1% BSA, 0,1% Tween 20) werden viermal mit 1 mL Waschpuffer (PBS pH 7,4, 0,1%BSA) gewaschen und anschließend 100 µL aus dem Kulturüberstand eines Ankerrezeptors (extrazelluläre TSH-Rezeptorchimäre N-terminal mit HSA fusioniert und C-terminal mit einem monoklonalen Antikörper fusioniert) zugegeben. Die entstandene Lösung wird unter Schütteln für 1 h bei Raumtemperatur inkubiert. Anschließend wird der Überstand abgenommen, die Dynabeads® zweimal mit 1 mL Waschpuffer gewaschen, in 30 µL Waschpuffer resuspendiert und bei 4°C gelagert.

### Beispiel 2 (Durchführung des erfindungsgemäßen Tests)

Serum- oder Plasmaproben werden innerhalb von 3 Stunden aus venösem Blut gewonnen. Lagerung bei 4°C über einen Zeitraum von 7 Tagen oder bei -20°C 1 bis 2 Jahre möglich. Sekundäre Antikörper (siehe oben) werden bei -20°C aufbewahrt und vor dem Einsatz im Test bei Raumtemperatur aufgetaut. TSH-Rezeptorchimären werden auf Mikrotiterplatten lyophilisiert gelagert und vor Gebrauch in Puffer mit Proteinstabilisatoren rekonstituiert.

Pro 2mL-Reaktionsgefäß werden 30 µL einer Lösung enthaltend 20 mg/mL mit Ankerrezeptor gekoppelte Dynabeads® in Waschpuffer (PBS pH 7,4, 0,1% BSA) mit 50 µL Waschpuffer verdünnt. Nach erfolgter Zugabe von 50 µL Serum oder Plasma der Patientenprobe wird das Reaktionsgefäß unter kräftigem Schütteln für 10 Minuten bei 37°C inkubiert. Anschließend erfolgen mehrere Waschschritte unter Aktivierung/Deaktivierung des Magneten wie zuvor beschrieben, mit jeweils 1 mL Waschpuffer. Nach erfolgter Zugabe von 100 µl einer 1:5 im Waschpuffer verdünnten Lösung eines Signalrezeptors (extrazelluläre TSH-Rezeptorchimäre N-terminal mit sekretorischer alkalischer Phosphatase (SEAP) fusioniert) wird das Reaktionsgefäß unter kräftigem Schütteln für 10 bis höchstens 20 Minuten bei 37°C inkubiert. Anschließend erfolgen mehrere Waschschritte mit jeweils 1 mL Waschpuffer, wie zuvor beschrieben. Danach erfolgt die Zugabe von 100 µl unverdünnter 1,2-Dioxetan enthaltenden Substrat-Lösung (AP-x (AB) von p·j·k. GmbH, Kleinblittersdorf, Deutschland) und das Reaktionsgefäß wird für 30 Minuten bei Raumtemperatur lichtgeschützt geschüttelt. Die Bio/Chemolumineszenz wird mit einem Röhrchenluminometer gemessen. Hierzu wird der Magnet aktiviert, die Lösung von den paramagnetischen Teilchen abgenommen und in ein Messröhrchen überführt. Das Messröhrchen wird in das Röhrchenluminometer gestellt und die Messung der Bio/Chemolumineszenz entsprechend den Anweisungen des Röhrchenluminometers durchgeführt.

### Verhalten einer sTRAb-DERA kalibrierten Standardreihe

Zur Erstellung der Standardreihe wurde eine Standardlösung WHO 90/672 mit einer standardisierten Konzentration von Autoantikörper eingesetzt. Die Ausgangslösung des Standards enthält 100 IU/l, die für die Zwecke der Erstellung der Standardkurve verdünnt wurden. Als Nullwert wurde das Serum eine Probanden ohne Autoantikörper in TXBW-Puffer verwendet.

Die eingesetzten TSH-Rezeptor-Chimären zum Binden von stimulierenden Autoantikörpern sind fixiert über Antikörper auf Dynabeads®, lyophilisiert gelagert und werden vor Gebrauch in Puffer mit Proteinstabilisatoren rekonstituiert. Der Triton X-100-Waschpuffer (TXWP) enthält 0,1% TX-100, 50 mM Tris/HCl pH 8,0,100 mM NaCl. Der Serumverdünnungspuffer enthält 5% Glucose und 5% Milchpulver in TXWP.

50 µl Probenlösung (Verdünnungen des Standards und Nullprobe) pro Vertiefung werden mit Verdünnungspuffer 1:2 verdünnt und 90 Minuten lang bei Raumtemperatur (etwa 22°C) auf Mikrotiterplatten inkubiert. Es wird viermal mit je 300 µl TXWP gewaschen. Dann erfolgt die Zugabe von 10µl einer 1:100-Verdünnung aus 5 ml Kulturüberstand einer Kulturschale eines Durchmessers von 10 cm von extrazellulärer TSH-Rezeptorchimäre, fusioniert mit einem Enzym SEAP, zu 90 µl TXWP. Anschließend wird unter Schütteln (300-400 UpM) 30 Minuten lang bei 37 °C inkubiert. Dann wird viermal mit 300 µl TXWP gewaschen. Die Bio/Chemolumineszenz wird mit dem Centrol® IA LB 296 Mikrotiterplatten-Meßgerät der Berthold GmbH, Bad Wildbad, Schwarzwald, Deutschland unter Einsatz von Tropix® (Reagenz für ECL (enhanced Chemolumineszenz) von Applied Biosystems, Foster City, CA, USA gemessen.

Die Nachweisgrenze liegt bei etwa 0,2 IU/l. Es besteht eine polynomische Funktion zwischen relativen Lichteinheiten (RLU=Relative Light Unit und den Konzentrationen des Standards für stimulierende Autoantikörper über einen Bereich, der bis mindestens 40 IU/l reicht.

### Beispiel 3 (Vergleichsbeispiel)

In diesem Beispiel wurden einerseits zwei trunkierte TSH-Reptorchimären und andererseits eine Holorezeptochimäre als Ankerrezeptor und eine trunkierte Rezeptorchimäre (ECD) als Signalrezeptor gemäß der EP 1 929 311 B1 verwendet. Die Ankerrezeptorchimäre war immobilisiert an der Kunststoffwand der Mikrotiterplatte.

Der Ankerantikörper (10,75 mM Citronensäure; 69 mM HEPES; 50 % Glycerol; pH 7,0) über Nacht bei 4 °C in Carbonatpuffer (100 mM NaCO3/NaHCO3 pH 9,6) als 10 µg/ml auf der Mikrotiterplatte (MTP 96 wells) inkubiert. Nach viermaligem Waschen (300 µl pro well) mit Assaypuffer (0,1 % Triton X-100; 50 mM Tris-HCl pH 8,0; 100 mM NaCl) erfolgt das Blockieren der freien MTP-Oberfläche für 1 Stunde bei 37 °C mit Blockierlösung (5 % Milchpulver; 5 % Glucose in Carbonatpuffer). Anschließend erfolgt die Beschichtung der festen Phase mit Ankerrezeptoren in Assaypuffer für 1 Stunde bei 20-24 °C unter Schütteln (300 rpm). Holorezeptorchimären werden als Zellextrakt (in 1 % Triton X-100; 150 mM NaCl; 50 mM Tris-HCl pH 8,0; Complete®-Protease-Inhibitoren) eingesetzt mit einer Konzentration an Gesamtprotein von 500 µg/ml (abhängig von Rezeptorchimäre bis 2000 µg/ml). Der Einsatz sekretierter ECD-Rezeptorchimären erfolgt als Zellkulturüberstand (in RPMI 1640; 10 % FCS; 1 % Penicillin / Streptomycin [10.000 U, 10.000 µg/ml]) mit einer Konzentration von 10-100 % in Assaypuffer nach klonabhängig definierter Produktion der Überstände. Viermaliges Waschen (300 µl pro well) mit Assaypuffer schließt sich an. Optional ist eine Lyophilisierungstrocknung der MTPs möglich. Dazu erfolgt das Waschen nach der Ankerrezeptorinkubation nur zweimal mit Assaypuffer gefolgt von zweimaligem Waschen mit Lyophilisierungspuffer (50 mM HEPES-NaOH pH 6,5; 0,1 % Triton X-100; 5 % Milchpulver; 3 % Karion). Der letzte Waschschritt erfolgt mit einer verlängerten Inkubationszeit der MTP (5-10 min). Die MTPs werden umgehend in der Stickstoffgasphase (-180 °C bis -130 °C) schockgefroren (Minimalinkubation 30 min) und anschließend lyophilisiert (0,011 mbar / -60 °C, 4-24 Stunden abhängig von Anzahl MTPs).

Die Inkubation des Patientenserums (gegebenenfalls nach Rekonstitution getrockneter MTPs für 5-10 min in Assaypuffer) erfolgt 1:2 in Assaypuffer verdünnt für 90 min bei 20-24 °C unter Schütteln (300 rpm). Zur Entfernung des ungebundenen Materials wird viermal mit Assaypuffer gewaschen. Die Inkubation des Detektionsrezeptors erfolgt in Assaypuffer für 30 min unter Schütteln (300 rpm), bei sekretierten ECD-Rezeptorchimären bei 37 C und bei Holorezeptorchimären bei 20-24 °C. Die eingesetzte Konzentration beträgt bei Zellextrakten von Holorezeptorchimären 1000 µg/ml Gesamtprotein (abhängig von Rezeptorchimäre bis 3000 µg/ml), bei sekretierten ECD-Rezeptorchimären abhängig vom Klon 10-100 % nach klonabhängig definierter Produktion der Überstände. Anschließend wird viermal mit je 300 µl Assaypuffer pro well gewaschen. Die Detektion erfolgt durch Zugabe von Substrat (135 µM CDP-Star bzw. alternativ AP-Substrat 1:1,5 [bis 3] in 1,0 M Diethanolamin pH 9,8; 0,5 mM MgCl2; 0,0001 % Xylencyanol) mit Inkubation für 30 min bei 20-24 °C unter Schütteln (300 rpm) und anschließender Quantifizierung im Mikrotiterplattenluminometer.

Stabilitätsmessungen der Detektions-Rezeptorchimären erfolgen durch Vorinkubation über verschiedene Zeiträume mit anschließendem Einsatz im Bridge-Assay, wobei die Anker-rezeptorchimäre wie in dem der Anmeldung zu Grunde liegenden Bridge-Assay behandelt wird, das heißt, quasi affintätschromatographisch auf der festen Phase gebunden ist

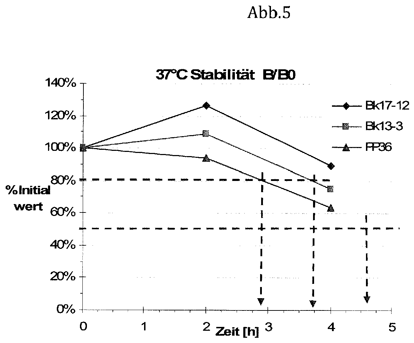

Bei den Messergebnissen des Vergleichs handelt es sich um Mikrotiterplatten-Versuche. Die Ergebnisse sind in Abbildung 5 gezeigt. Die Mikrotiterplatten wurden bei 4 °C mit einem Antikörper beschichtet, an den die Ankerrezeptorchimäre (Holorezeptorchimäre oder ECD-Rezeptorchimäre (Vergleich)) gebunden wurde. Die Inkubation mit Patientenserum erfolgte bei Raumtemperatur.

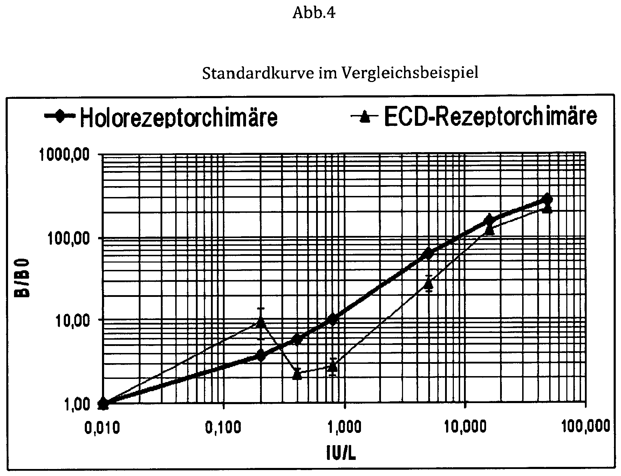

Bei den ermittelten Kurven der Messwerte in Abb. 4 handelt sich letztendlich um zwei Kalibrierungskurven, wobei der auf der Ordinate aufgetragene Quotient B/B0 (gebunden/ungebunden) verwendet wurde, um unspezifische Bindung im Ergebnis auszuschalten. Auf der Abzisse sind die Autoantikörpereinheiten einer standardisierten Probe angegeben.

Die fettgedruckte Kurve zeigt die Ausführungsform mit einer an eine Festphase gebundenen ersten TSH-Rezeptorchimäre (Holorezeptorchimäre) und einer zweiten trunkierten TSH-Rezeptorchimäre (ECD-Rezeptorchimäre), die nur den extrazellulären Teil des Holorezeptors umfasst als Signalrezeptor. Im Falle der dünn gedruckten Kurve wird als Ankerrezeptorchimäre anstatt der an eine Festphase gebundenen ersten TSH-Rezeptorchimäre (Holorezeptorchimäre) ebenfalls die trunkierte Rezeptorchimäre (ECD-Rezeptorchimäre) eingesetzt.

Der Vergleich der beiden Kurvenverläufe zeigt deutliche Unterschiede im Bindungsverhalten der Autoantikörper an die Holo- bzw. trunkierten Rezeptorchimären. Die Kurve der Messwerte der Proben mit zwei ECD-Rezeptorchimären (Anker- und Signalrezeptor sind ECD-Rezeptorchimäre) "zackelt", ist nicht reproduzierbar und liefert nicht die benötigte Sensitivität und Spezifität. Sie ist insbesondere ungeeignet für einen breiten Konzentrationsbereich. Der Einsatz einer Holorezeptorchimäre als Ankerrezeptor und einer weiteren Holorezeptorchimäre eignet sich folglich weder für Mikrotiterplatten-Versuche noch für die Bestimmung auf einem Automaten.

## Patentansprüche

1. Auf einem Diagnose-Automaten durchführbares in vitro Verfahren zum Nachweis von stimulierenden Autoantikörpern gegen den TSH-Rezeptor, in dem man eine Patientenprobe in Kontakt bringt mit einer an eine Festphase gebundene erste TSH-Rezeptorchimäre, um in der Patientenprobe gegebenenfalls vorhandene stimulierende Autoantikörper gegen den TSH-Rezeptor an der ersten TSH-Rezeptorchimäre zu binden, einen erhaltenen Immunkomplex mit einer zweiten TSH-Rezeptorchimäre reagieren lässt, um in der Patientenprobe gegebenenfalls vorhandene stimulierende Autoantikörper gegen den TSH-Rezeptor zusätzlich an der zweiten TSH-Rezeptorchimäre zu binden und eine Reaktion zum Nachweis des den Autoantikörper enthaltenden Immunkomplexes durchführt, wobei beide TSH-Rezeptorchimären keine Bindungsepitope für neutrale und blockierende Autoantikörper aber das Bindungsepitop für stimulierende Autoantikörper gegen den TSH-Rezeptor aufweisen, **dadurch gekennzeichnet, dass** man als Festphase paramagnetische Teilchen einsetzt, die erste und die zweite TSH-Rezeptorchimäre nur den extrazellulären Teil der TSH-Rezeptorchimären umfassen, die TSH-Rezeptorchimären N-terminal mit einem zur Sezernierung der TSH-Rezeptorchimäre in den Extrazellulärraum geeigneten Protein fusioniert sind, das zur Sezernierung der ersten und der zweiten TSH-Rezeptorchimäre in den Extrazellulärraum geeignete Protein ein Serumprotein ist und die zweite TSH-Rezeptorchimäre eine signalgebende Aminosäuresequenz oder ein anderes signalgebendes Markierungsmittel zum Nachweis des gebildeten Immunkomplexes aufweist und die TSH-Rezeptorchimären die Aminosäuresequenz (SEQ ID Nr. 4) aufweisen:

2. Verfahren nach Anspruch 1, **dadurch gekennzeichnet, dass** die erste und/oder die zweite TSH-Rezeptorchimäre N-terminal mit humanem Serumprotein, insbesondere Albumin, fusioniert ist.

3. Verfahren nach einem der Ansprüche 1 oder 2, **dadurch gekennzeichnet, dass** die zweite TSH-Rezeptorchimäre N-terminal mit sekretorischer alkalischer Phosphatase fusioniert ist.

4. Verfahren nach einem der Ansprüche 1 bis 3, **dadurch gekennzeichnet, dass** das signalgebende Markierungsmittel sekretorische alkalische Phosphatase, eine Leuchtkäfer- oder Gausialuciferase oder eine Peroxidase ist.

5. Verfahren nach einem der Ansprüche 1, **dadurch gekennzeichnet, dass** das signalgebende Markierungsmittel eine Acridin- oder Rutheniumverbindung oder Fluoresceinisothiocyanatverbindung ist.

## Claims

1. An in vitro method for the detection of stimulating autoantibodies against the TSH receptor, which can be performed on an automatic diagnostic apparatus, in which a patient sample is contacted with a first TSH receptor chimera bound to a solid phase in order to bind any stimulating autoantibodies against the TSH receptor present in the patient sample to the first TSH receptor chimera, reacting an obtained immune complex with a second TSH receptor chimera in order to additionally bind any stimulating autoantibodies against the TSH receptor present in the patient sample to the second TSH receptor chimera and carrying out a reaction to detect the immune complex containing the autoantibody, wherein both TSH receptor chimeras have no binding epitopes for neutral and blocking autoantibodies but the binding epitope for stimulating autoantibodies against the TSH receptor, **characterized in that** paramagnetic particles are used as solid phase, the first and second TSH receptor chimeras comprising only the extracellular part of the TSH receptor chimeras, the TSH receptor chimera N-terminal are fused with a protein suitable for the secretion of the TSH receptor chimera into the extracellular space, the protein suitable for secreting the first and second TSH receptor chimeras into the extracellular space is a serum protein, and the second TSH receptor chimera comprises a signaling amino acid sequence or other signaling label for detecting the formed immune complex, and the TSH receptor chimeras comprise the amino acid sequence (SEQ ID No. 4):

2. The method as claimed in claim 1, **characterized in that** the first and/or the second TSH receptor chimera is N-terminally fused with human serum protein, in particular albumin.

3. The method as claimed in one of claims 1 or 2, **characterized in that** the second TSH receptor chimera is N-terminally fused with secretory alkaline phosphatase.

4. The method as claimed in one of claims 1 to 3, **characterized in that** the signal-emitting marker is secretory alkaline phosphatase, a firefly or Gaussia luciferase or a peroxidase.

5. The method as claimed in one of claims 1, **characterized in that** the signal-emitting marker is an acridine or ruthenium compound or fluorescein isothiocyanate compound.

## Revendications

1. Procédé in vitro réalisable sur une machine de diagnostic, pour la détection d'auto-anticorps stimulants contre le récepteur de TSH, dans lequel un échantillon de patient est mis en contact avec une première chimère du récepteur de TSH liée à une phase solide pour lier l'auto-anticorps stimulant contre le récepteur de TSH, éventuellement présent dans l'échantillon de patient à la première chimère du récepteur de TSH, un premier complexe immun obtenu est mis à réagir avec une deuxième chimère du récepteur de TSH pour également lier l'auto-anticorps stimulant contre le récepteur de TSH, éventuellement présent dans l'échantillon de patient à la deuxième chimère du récepteur de TSH, et effectuer une réaction pour la détection du complexe immun contenant l'auto-anticorps, où les deux chimères du récepteur de TSH ne présentent aucun épitope de liaison pour les auto-anticorps neutre et bloquant, mais bien l'épitope de liaison pour l'auto-anticorps stimulant contre le récepteur de TSH, **caractérisé en ce que** l'on utilise des particules paramagnétiques comme phase solide, la première et la deuxième chimère du récepteur de TSH ne comprennent que la partie extracellulaire des chimères du récepteur de TSH, les chimères du récepteur de TSH étant fusionnées sur l'extrémité N à une protéine appropriée pour la sécrétion de la chimère du récepteur de TSH dans l'espace extracellulaire, la protéine appropriée pour la sécrétion de la première et de la deuxième chimère du récepteur de TSH dans l'espace extracellulaire étant une protéine sérique, et la deuxième chimère du récepteur de TSH présentant une séquence des acides aminés émettant un signal ou un autre agent de marquage émettant un signal pour la détection du complexe immun formé et les chimères du récepteur de TSH présentant la séquence des acides aminés (SEQ ID N°4) :

2. Procédé selon la revendication 1, **caractérisé en ce que** la première et/ou la deuxième chimère du récepteur de TSH est fusionnée sur l'extrémité N à une protéine sérique humaine, en particulier l'albumine.

3. Procédé selon la revendication 1 ou 2, **caractérisé en ce que** la deuxième chimère du récepteur de la TSH est fusionnée à l'extrémité N à la phosphatase alcaline sécrétoire.

4. Procédé selon l'une quelconque des revendications 1 à 3, **caractérisé en ce que** l'agent de marquage émettant un signal est une phosphatase alcaline sécrétoire, une luciférase de luciole ou de Gaussia.

5. Procédé selon la revendication 1, **caractérisé en ce que** l'agent de marquage émettant un signal est un composé d'acridine ou de ruthénium ou un composé d'isothiocyanate de fluorescéine.


### SEQUENZ LISTING

<110> Loos, Ulrich
<120> Nachweis von Autoimmunantikörpern gegen den TSH-Rezeptor
<130> 111270DE
<160> 7
<170> PatentIn version 3.5
<210> 1
   <211> 1728
   <212> DNA
   <213> Artificial Sequence
<220>
   <223> HSA2-TSHR-ECD-B330-mab1 with untranslated part
<400> 1
<210> 2
   <211> 566
   <212> PRT
   <213> Artificial Sequence
<220>
   <223> HSA2-TSHR-ECD-B330-mab1 protein
<400> 2
<210> 3
   <211> 240
   <212> PRT
   <213> Artificial Sequence
<220>
   <223> amino acids 21-260 ECD of TSHR (ECD260)
<400> 3
   Gly Met Gly Cys Ser Ser Pro Pro Cys Glu Cys His Gln Glu Glu Asp
   1               5                   10                  15
   Phe Arg Val Thr Cys Lys Asp Ile Gln Arg Ile Pro Ser Leu Pro Pro
               20                  25                  30
   Ser Thr Gln Thr Leu Lys Leu Ile Glu Thr His Leu Arg Thr Ile Pro
           35                  40                  45
   Ser His Ala Phe Ser Asn Leu Pro Asn Ile Ser Arg Ile Tyr Val Ser
       50                  55                  60
   Ile Asp Val Thr Leu Gln Gln Leu Glu Ser His Ser Phe Tyr Asn Leu
   65                  70                  75                  80
   Ser Lys Val Thr His Ile Glu Ile Arg Asn Thr Arg Asn Leu Thr Tyr
                   85                  90                  95
   Ile Asp Pro Asp Ala Leu Lys Glu Leu Pro Leu Leu Lys Phe Leu Gly
               100                 105                 110
   Ile Phe Asn Thr Gly Leu Lys Met Phe Pro Asp Leu Thr Lys Val Tyr
           115                 120                 125
   Ser Thr Asp Ile Phe Phe Ile Leu Glu Ile Thr Asp Asn Pro Tyr Met
       130                 135                 140
   Thr Ser Ile Pro Val Asn Ala Phe Gln Gly Leu Cys Asn Glu Thr Leu
   145                 150                 155                 160
   Thr Leu Lys Leu Tyr Asn Asn Gly Phe Thr Ser Val Gln Gly Tyr Ala
                   165                 170                 175
   Phe Asn Gly Thr Lys Leu Asp Ala Val Tyr Leu Asn Lys Asn Lys Tyr
               180                 185                 190
   Leu Thr Val Ile Asp Lys Asp Ala Phe Gly Gly Val Tyr Ser Gly Pro
           195                 200                 205
   Ser Leu Leu Asp Val Ser Gln Thr Ser Val Thr Ala Leu Pro Ser Lys
       210                 215                 220
   Gly Leu Glu His Leu Lys Glu Leu Ile Ala Arg Asn Thr Trp Thr Leu
   225                 230                 235                 240
<210> 4
   <211> 310
   <212> PRT
   <213> Artificial Sequence
<220>
   <223> amino acids 21-330 ECD of TSHR (ECD330)
<400> 4
<110> Ulrich Loos
<120> Neuer Brückenassay
<130> 111270EP
<160> 2
<170> BiSSAP 1.2
<210> 1
   <211> 2012
   <212> DNA
   <213> Artificial Sequence
<220>
   <221> source
   <222> 1..2012
   <223> /mol_type="unassigned DNA" /note="Wildtyp-ECD des TSH-Fusionsproteins" /organism="Artificial Sequence"
<400> 1
<210> 2
   <211> 652
   <212> PRT
   <213> Artificial Sequence
<220>
   <223> TSHR Wildtyp ECD
<400> 2
   Met Lys Trp Val Thr Phe Ile Ser Leu Leu Phe Leu Phe Ser Ser Ala
   1               5                   10                  15
   Tyr Ser Arg Gly Val Phe Arg Arg Asp Ala His Lys Ser Glu Val Ala
               20                  25                  30
   His Arg Phe Lys Asp Leu Gly Glu Glu Asn Phe Lys Ala Leu Val Leu
           35                  40                  45
   Ile Ala Phe Ala Gln Tyr Leu Gln Gln Cys Pro Phe Glu Asp His Val
       50                  55                  60
   Lys Leu Val Asn Glu Val Thr Glu Phe Ala Lys Thr Cys Val Ala Asp
   65                  70                  75                  80
   Glu Ser Ala Glu Asn Cys Asp Lys Ser Leu His Thr Leu Phe Gly Asp
                   85                  90                  95
   Lys Leu Cys Thr Val Ala Thr Leu Arg Glu Thr Tyr Gly Glu Met Ala
               100                 105                 110
   Asp Cys Cys Ala Lys Gln Glu Pro Glu Arg Asn Glu Cys Phe Leu Gln
           115                 120                 125
   His Lys Asp Asp Asn Pro Asn Leu Pro Arg Leu Val Arg Pro Glu Val
       130                 135                 140
   Asp Val Met Cys Thr Ala Phe His Asp Asn Glu Glu Thr Phe Leu Lys
   145                 150                 155                 160
   Lys Tyr Leu Tyr Glu Ile Ala Arg Arg His Pro Tyr Phe Tyr Ala Pro
                   165                 170                 175
   Glu Leu Leu Phe Phe Ala Lys Arg Tyr Lys Ala Ala Phe Thr Glu Cys
               180                 185                 190
   Cys Gln Ala Ala Asp Lys Ala Ala Cys Leu Leu Pro Lys Leu Met Arg
           195                 200                 205
   Pro Ala Asp Leu Leu Gln Leu Val Leu Leu Leu Asp Leu Pro Arg Asp
       210                 215                 220
   Leu Gly Gly Met Gly Cys Ser Ser Pro Pro Cys Glu Cys His Gln Glu
   225                 230                 235                 240
   Glu Asp Phe Arg Val Thr Cys Lys Asp Ile Gln Arg Ile Pro Ser Leu
                   245                 250                 255
   Pro Pro Ser Thr Gln Thr Leu Lys Leu Ile Glu Thr His Leu Arg Thr
               260                 265                 270
   Ile Pro Ser His Ala Phe Ser Asn Leu Pro Asn Ile Ser Arg Ile Tyr
           275                 280                 285
   Val Ser Ile Asp Val Thr Leu Gln Gln Leu Glu Ser His Ser Phe Tyr
       290                 295                 300
   Asn Leu Ser Lys Val Thr His Ile Glu Ile Arg Asn Thr Arg Asn Leu
   305                 310                 315                 320
   Thr Tyr Ile Asp Pro Asp Ala Leu Lys Glu Leu Pro Leu Leu Lys Phe
                   325                 330                 335
   Leu Gly Ile Phe Asn Thr Gly Leu Lys Met Phe Pro Asp Leu Thr Lys
               340                 345                 350
   Val Tyr Ser Thr Asp Ile Phe Phe Ile Leu Glu Ile Thr Asp Asn Pro
           355                 360                 365
   Tyr Met Thr Ser Ile Pro Val Asn Ala Phe Gln Gly Leu Cys Asn Glu
       370                 375                 380
   Thr Leu Thr Leu Lys Leu Tyr Asn Asn Gly Phe Thr Ser Val Gln Gly
   385                 390                 395                 400
   Tyr Ala Phe Asn Gly Thr Lys Leu Asp Ala Val Tyr Leu Asn Lys Asn
                   405                 410                 415
   Lys Tyr Leu Thr Val Ile Asp Lys Asp Ala Phe Gly Gly Val Tyr Ser
               420                 425                 430
   Gly Pro Ser Leu Leu Asp Val Ser Gln Thr Ser Val Thr Ala Leu Pro
           435                 440                 445
   Ser Lys Gly Leu Glu His Leu Lys Glu Leu Ile Ala Arg Asn Thr Trp
       450                 455                 460
   Thr Leu Lys Lys Leu Pro Leu Ser Leu Ser Phe Leu His Leu Thr Arg
   465                 470                 475                 480
   Ala Asp Leu Ser Tyr Pro Ser His Cys Cys Ala Phe Lys Asn Gln Lys
                   485                 490                 495
   Lys Ile Arg Gly Ile Leu Glu Ser Leu Met Cys Asn Glu Ser Ser Met
               500                 505                 510
   Gln Ser Leu Arg Gln Arg Lys Ser Val Asn Ala Leu Asn Ser Pro Leu
           515                 520                 525
   His Gln Glu Tyr Glu Glu Asn Leu Gly Asp Ser Ile Val Gly Tyr Lys
       530                 535                 540
   Glu Lys Ser Lys Phe Gln Asp Thr His Asn Asn Ala His Tyr Tyr Val
   545                 550                 555                 560
   Phe Phe Glu Glu Gln Glu Asp Glu Ile Ile Gly Phe Gly Gln Glu Leu
                   565                 570                 575
   Lys Asn Pro Gln Glu Glu Thr Leu Gln Ala Phe Asp Ser His Tyr Asp
               580                 585                 590
   Tyr Thr Ile Cys Gly Asp Ser Glu Asp Met Val Cys Thr Pro Lys Ser
           595                 600                 605
   Asp Glu Phe Asn Pro Cys Glu Asp Ile Met Gly Tyr Lys Phe Leu Arg
       610                 615                 620
   Ile Cys Lys Arg Gln Ala Gln Ala Tyr Arg Gly Gln Arg Val Pro Pro
   625                 630                 635                 640
   Lys Asn Ser Thr Asp Ile Gln Val Gln Lys Val Thr
                   645                 650

### SEQUENCE LISTING

<110> Ulrich Loos
<120> Nachweis Autoantikörper gegen TSHR
<130> 111270EP
<160> 6
<170> BiSSAP 1.2
<210> 1
   <211> 1728
   <212> DNA
   <213> Artificial Sequence
<220>
   <221> source
   <222> 1..1728
   <223> /mol_type="unassigned DNA" /note="Sequenz 1" /organism="Artificial Sequence"
<400> 1
   gatatcctag cttttctctt ctgtcaaccc cacacgcctt tggcacagat atcacaatga 60
   agtgggtaac ctttatttcc cttctttttc tctttagctc ggcttattcc aggggtgtgt 120
   ttcgtcgaga tgcacacaag agtgaggttg ctcatcggtt taaagatttg ggagaagaaa 180
   atttcaaagc cttggtgttg attgcctttg ctcagtatct tcagcagtgt ccatttgaag 240
   atcatgtaaa attagtgaat gaagtaactg aatttgcaaa aacatgtgtt gctgatgagt 300
   cagctgaaaa ttgtgacaaa tcacttcata ccctttttgg agacaaatta tgcacagttg 360
   caactcttcg tgaaacctat ggtgaaatgg ctgactgctg tgcaaaacaa gaacctgaga 420
   gaaatgaatg cttcttgcaa cacaaagatg acaacccaaa cctcccccga ttggtgagac 480
   cagaggttga tgtgatgtgc actgcttttc atgacaatga agagacattt ttgaaaaaat 540
   acttatatga aattgccaga agacatcctt acttttatgc cccggaactc cttttctttg 600
   ctaaaaggta taaagctgct tttacagaat gttgccaagc tgctgataaa gctgcctgcc 660
   tgttgccaaa gctcactagt ggaatggggt gttcgtctcc accctgcgag tgccatcagg 720
   aggaggactt cagagtcacc tgcaaggata ttcaacgcat ccccagctta ccgcccagta 780
   cgcagactct gaagcttatt gagactcacc tgagaactat tccaagtcat gcattttcta 840
   atctgcccaa tatttccaga atctacgtat ctatagatgt gactctgcag cagctggaat 900
   cacactcctt ctacaatttg agtaaagtga ctcacataga aattcggaat accaggaact 960
   taacttacat agaccctgat gccctcaaag agctccccct cctaaagttc cttggcattt 1020
   tcaacactgg acttaaaatg ttccctgacc tgaccaaagt ttattccact gatatattct 1080
   ttatacttga aattacagac aacccttaca tgacgtcaat ccctgtgaat gcttttcagg 1140
   gactatgcaa tgaaaccttg acactgaagc tgtacaacaa tggctttact tcagtccaag 1200
   gatatgcttt caatgggaca aagctggatg ctgtttacct aaacaagaat aaatacctga 1260
   cagttattga caaagatgca tttggaggag tatacagtgg accaagcttg ctggacgtgt 1320
   ctcaaaccag tgtcactgcc cttccatcca aaggcctgga gcacctgaag gaactgatag 1380
   caagaaacac ctggactctt aagaaaacac tgccctccaa agaaaaattc acgagcctcc 1440
   tggtcgccac gctgacctac cccagccact gctgcgcctt caggaatttg ccgaagaaag 1500
   aacagaattt ttcattttcc atttttgaaa acttctccaa acaatgcgaa agcacagtta 1560
   gaaaagcaga taacgagacg ctttattccg ccatctttga ggagaatgaa gctagcatct 1620
   gtaaacgcca ggctcaggca taccgggggc agagggttcc tccaaagaac agcactgata 1680
   ttcaggttca aaaggttacc taaggatcca ctagtcagtg tgctggaa 1728
<210> 2
   <211> 1570
   <212> PRT
   <213> Artificial Sequence
<220>
   <223> Sequenz 2
<400> 2
   Thr Tyr Arg Pro Arg Ser Glu Arg Pro His Glu Ser Glu Arg Leu Glu
   1               5                   10                  15
   Leu Glu Ser Glu Arg Thr His Arg Pro Arg His Ile Ser Ala Leu Ala
               20                  25                  30
   Pro His Glu Gly Leu Tyr Thr His Arg Ala Ser Pro Ile Leu Glu Thr
           35                  40                  45
   His Arg Met Glu Thr Leu Tyr Ser Thr Arg Pro Val Ala Leu Thr His
       50                  55                  60
   Arg Pro His Glu Ile Leu Glu Ser Glu Arg Leu Glu Leu Glu Pro His
   65                  70                  75                  80
   Glu Leu Glu Pro His Glu Ser Glu Arg Ser Glu Arg Ala Leu Ala Thr
                   85                  90                  95
   Tyr Arg Ser Glu Arg Ala Arg Gly Gly Leu Tyr Val Ala Leu Pro His
               100                 105                 110
   Glu Ala Arg Gly Ala Arg Gly Ala Ser Pro Ala Leu Ala His Ile Ser
           115                 120                 125
   Leu Tyr Ser Ser Glu Arg Gly Leu Val Ala Leu Ala Leu Ala His Ile
       130                 135                 140
   Ser Ala Arg Gly Pro His Glu Leu Tyr Ser Ala Ser Pro Leu Glu Gly
   145                 150                 155                 160
   Leu Tyr Gly Leu Gly Leu Ala Ser Asn Pro His Glu Leu Tyr Ser Ala
                   165                 170                 175
   Leu Ala Leu Glu Val Ala Leu Leu Glu Ile Leu Glu Ala Leu Ala Pro
               180                 185                 190
   His Glu Ala Leu Ala Gly Leu Asn Thr Tyr Arg Leu Glu Gly Leu Asn
           195                 200                 205
   Gly Leu Asn Cys Tyr Ser Pro Arg Pro His Glu Gly Leu Ala Ser Pro
       210                 215                 220
   His Ile Ser Val Ala Leu Leu Tyr Ser Leu Glu Val Ala Leu Ala Ser
   225                 230                 235                 240
   Asn Gly Leu Val Ala Leu Thr His Arg Gly Leu Pro His Glu Ala Leu
                   245                 250                 255
   Ala Leu Tyr Ser Thr His Arg Cys Tyr Ser Val Ala Leu Ala Leu Ala
               260                 265                 270
   Ala Ser Pro Gly Leu Ser Glu Arg Ala Leu Ala Gly Leu Ala Ser Asn
           275                 280                 285
   Cys Tyr Ser Ala Ser Pro Leu Tyr Ser Ser Glu Arg Leu Glu His Ile
       290                 295                 300
   Ser Thr His Arg Leu Glu Pro His Glu Gly Leu Tyr Ala Ser Pro Leu
   305                 310                 315                 320
   Tyr Ser Leu Glu Cys Tyr Ser Thr His Arg Val Ala Leu Ala Leu Ala
                   325                 330                 335
   Thr His Arg Leu Glu Ala Arg Gly Gly Leu Thr His Arg Thr Tyr Arg
               340                 345                 350
   Gly Leu Tyr Gly Leu Met Glu Thr Ala Leu Ala Ala Ser Pro Cys Tyr
           355                 360                 365
   Ser Cys Tyr Ser Ala Leu Ala Leu Tyr Ser Gly Leu Asn Gly Leu Pro
       370                 375                 380
   Arg Gly Leu Ala Arg Gly Ala Ser Asn Gly Leu Cys Tyr Ser Pro His
   385                 390                 395                 400
   Glu Leu Glu Gly Leu Asn His Ile Ser Leu Tyr Ser Ala Ser Pro Ala
                   405                 410                 415
   Ser Pro Ala Ser Asn Pro Arg Ala Ser Asn Leu Glu Pro Arg Ala Arg
               420                 425                 430
   Gly Leu Glu Val Ala Leu Ala Arg Gly Pro Arg Gly Leu Val Ala Leu
           435                 440                 445
   Ala Ser Pro Val Ala Leu Met Glu Thr Cys Tyr Ser Thr His Arg Ala
       450                 455                 460
   Leu Ala Pro His Glu His Ile Ser Ala Ser Pro Ala Ser Asn Gly Leu
   465                 470                 475                 480
   Gly Leu Thr His Arg Pro His Glu Leu Glu Leu Tyr Ser Leu Tyr Ser
                   485                 490                 495
   Thr Tyr Arg Leu Glu Thr Tyr Arg Gly Leu Ile Leu Glu Ala Leu Ala
               500                 505                 510
   Ala Arg Gly Ala Arg Gly His Ile Ser Pro Arg Thr Tyr Arg Pro His
           515                 520                 525
   Glu Thr Tyr Arg Ala Leu Ala Pro Arg Gly Leu Leu Glu Leu Glu Pro
       530                 535                 540
   His Glu Pro His Glu Ala Leu Ala Leu Tyr Ser Ala Arg Gly Thr Tyr
   545                 550                 555                 560
   Arg Leu Tyr Ser Ala Leu Ala Ala Leu Ala Pro His Glu Thr His Arg
                   565                 570                 575
   Gly Leu Cys Tyr Ser Cys Tyr Ser Gly Leu Asn Ala Leu Ala Ala Leu
               580                 585                 590
   Ala Ala Ser Pro Leu Tyr Ser Ala Leu Ala Ala Leu Ala Cys Tyr Ser
           595                 600                 605
   Leu Glu Leu Glu Pro Arg Leu Tyr Ser Leu Glu Thr His Arg Ser Glu
       610                 615                 620
   Arg Gly Leu Tyr Met Glu Thr Gly Leu Tyr Cys Tyr Ser Ser Glu Arg
   625                 630                 635                 640
   Ser Glu Arg Pro Arg Pro Arg Cys Tyr Ser Gly Leu Cys Tyr Ser His
                   645                 650                 655
   Ile Ser Gly Leu Asn Gly Leu Gly Leu Ala Ser Pro Pro His Glu Ala
               660                 665                 670
   Arg Gly Val Ala Leu Thr His Arg Cys Tyr Ser Leu Tyr Ser Ala Ser
           675                 680                 685
   Pro Ile Leu Glu Gly Leu Asn Ala Arg Gly Ile Leu Glu Pro Arg Ser
       690                 695                 700
   Glu Arg Leu Glu Pro Arg Pro Arg Ser Glu Arg Thr His Arg Gly Leu
   705                 710                 715                 720
   Asn Thr His Arg Leu Glu Leu Tyr Ser Leu Glu Ile Leu Glu Gly Leu
                   725                 730                 735
   Thr His Arg His Ile Ser Leu Glu Ala Arg Gly Thr His Arg Ile Leu
               740                 745                 750
   Glu Pro Arg Ser Glu Arg His Ile Ser Ala Leu Ala Pro His Glu Ser
           755                 760                 765
   Glu Arg Ala Ser Asn Leu Glu Pro Arg Ala Ser Asn Ile Leu Glu Ser
   770 775 780
   Glu Arg Ala Arg Gly Ile Leu Glu Thr Tyr Arg Val Ala Leu Ser Glu
   785 790 795 800
   Arg Ile Leu Glu Ala Ser Pro Val Ala Leu Thr His Arg Leu Glu Gly
   805 810 815
   Leu Asn Gly Leu Asn Leu Glu Gly Leu Ser Glu Arg His Ile Ser Ser
   820 825 830
   Glu Arg Pro His Glu Thr Tyr Arg Ala Ser Asn Leu Glu Ser Glu Arg
   835 840 845
   Leu Tyr Ser Val Ala Leu Thr His Arg His Ile Ser Ile Leu Glu Gly
   850 855 860
   Leu Ile Leu Glu Ala Arg Gly Ala Ser Asn Thr His Arg Ala Arg Gly
   865 870 875 880
   Ala Ser Asn Leu Glu Thr His Arg Thr Tyr Arg Ile Leu Glu Ala Ser
   885 890 895
   Pro Pro Arg Ala Ser Pro Ala Leu Ala Leu Glu Leu Tyr Ser Gly Leu
   900 905 910
   Leu Glu Pro Arg Leu Glu Leu Glu Leu Tyr Ser Pro His Glu Leu Glu
   915 920 925
   Gly Leu Tyr Ile Leu Glu Pro His Glu Ala Ser Asn Thr His Arg Gly
   930 935 940
   Leu Tyr Leu Glu Leu Tyr Ser Met Glu Thr Pro His Glu Pro Arg Ala
   945 950 955 960
   Ser Pro Leu Glu Thr His Arg Leu Tyr Ser Val Ala Leu Thr Tyr Arg
   965 970 975
   Ser Glu Arg Thr His Arg Ala Ser Pro Ile Leu Glu Pro His Glu Pro
   980 985 990
   His Glu Ile Leu Glu Leu Glu Gly Leu Ile Leu Glu Thr His Arg Ala
   995 1000 1005
   Ser Pro Ala Ser Asn Pro Arg Thr Tyr Arg Met Glu Thr Thr His Arg
   1010 1015 1020
   Ser Glu Arg Ile Leu Glu Pro Arg Val Ala Leu Ala Ser Asn Ala Leu
   1025 1030 1035 1040
   Ala Pro His Glu Gly Leu Asn Gly Leu Tyr Leu Glu Cys Tyr Ser Ala
   1045 1050 1055
   Ser Asn Gly Leu Thr His Arg Leu Glu Thr His Arg Leu Glu Leu Tyr
   1060 1065 1070
   Ser Leu Glu Thr Tyr Arg Ala Ser Asn Ala Ser Asn Gly Leu Tyr Pro
   1075 1080 1085
   His Glu Thr His Arg Ser Glu Arg Val Ala Leu Gly Leu Asn Gly Leu
   1090 1095 1100
   Tyr Thr Tyr Arg Ala Leu Ala Pro His Glu Ala Ser Asn Gly Leu Tyr
   1105 1110 1115 1120
   Thr His Arg Leu Tyr Ser Leu Glu Ala Ser Pro Ala Leu Ala Val Ala
   1125 1130 1135
   Leu Thr Tyr Arg Leu Glu Ala Ser Asn Leu Tyr Ser Ala Ser Asn Leu
   1140 1145 1150
   Tyr Ser Thr Tyr Arg Leu Glu Thr His Arg Val Ala Leu Ile Leu Glu
   1155 1160 1165
   Ala Ser Pro Leu Tyr Ser Ala Ser Pro Ala Leu Ala Pro His Glu Gly
   1170 1175 1180
   Leu Tyr Gly Leu Tyr Val Ala Leu Thr Tyr Arg Ser Glu Arg Gly Leu
   1185 1190 1195 1200
   Tyr Pro Arg Ser Glu Arg Leu Glu Leu Glu Ala Ser Pro Val Ala Leu
   1205 1210 1215
   Ser Glu Arg Gly Leu Asn Thr His Arg Ser Glu Arg Val Ala Leu Thr
   1220 1225 1230
   His Arg Ala Leu Ala Leu Glu Pro Arg Ser Glu Arg Leu Tyr Ser Gly
   1235 1240 1245
   Leu Tyr Leu Glu Gly Leu His Ile Ser Leu Glu Leu Tyr Ser Gly Leu
   1250 1255 1260
   Leu Glu Ile Leu Glu Ala Leu Ala Ala Arg Gly Ala Ser Asn Thr His
   1265                1270                1275                1280
   Arg Thr Arg Pro Thr His Arg Leu Glu Leu Tyr Ser Leu Tyr Ser Thr
                   1285                1290                1295
   His Arg Leu Glu Pro Arg Ser Glu Arg Leu Tyr Ser Gly Leu Leu Tyr
               1300                1305                1310
   Ser Pro His Glu Thr His Arg Ser Glu Arg Leu Glu Leu Glu Val Ala
           1315                1320                1325
   Leu Ala Leu Ala Thr His Arg Leu Glu Thr His Arg Thr Tyr Arg Pro
       1330                1335                1340
   Arg Ser Glu Arg His Ile Ser Cys Tyr Ser Cys Tyr Ser Ala Leu Ala
   1345                1350                1355                1360
   Pro His Glu Ala Arg Gly Ala Ser Asn Leu Glu Pro Arg Leu Tyr Ser
                   1365                1370                1375
   Leu Tyr Ser Gly Leu Gly Leu Asn Ala Ser Asn Pro His Glu Ser Glu
               1380                1385                1390
   Arg Pro His Glu Ser Glu Arg Ile Leu Glu Pro His Glu Gly Leu Ala
           1395                1400                1405
   Ser Asn Pro His Glu Ser Glu Arg Leu Tyr Ser Gly Leu Asn Cys Tyr
       1410                1415                1420
   Ser Gly Leu Ser Glu Arg Thr His Arg Val Ala Leu Ala Arg Gly Leu
   1425                1430                1435                1440
   Tyr Ser Ala Leu Ala Ala Ser Pro Ala Ser Asn Gly Leu Thr His Arg
                   1445                1450                1455
   Leu Glu Thr Tyr Arg Ser Glu Arg Ala Leu Ala Ile Leu Glu Pro His
               1460                1465                1470
   Glu Gly Leu Gly Leu Ala Ser Asn Gly Leu Ala Leu Ala Ser Glu Arg
           1475                1480                1485
   Ile Leu Glu Cys Tyr Ser Leu Tyr Ser Ala Arg Gly Gly Leu Asn Ala
       1490                1495                1500
   Leu Ala Gly Leu Asn Ala Leu Ala Thr Tyr Arg Ala Arg Gly Gly Leu
   1505                1510                1515                1520
   Tyr Gly Leu Asn Ala Arg Gly Val Ala Leu Pro Arg Pro Arg Leu Tyr
                   1525                1530                1535
   Ser Ala Ser Asn Ser Glu Arg Thr His Arg Ala Ser Pro Ile Leu Glu
               1540                1545                1550
   Gly Leu Asn Val Ala Leu Gly Leu Asn Leu Tyr Ser Val Ala Leu Thr
           1555                1560                1565
   His Arg
       1570
<210> 3
   <211> 664
   <212> PRT
   <213> Artificial Sequence
<220>
   <223> Sequenz 3
<400> 3
   Gly Leu Tyr Met Glu Thr Gly Leu Tyr Cys Tyr Ser Ser Glu Arg Ser
   1               5                   10                  15
   Glu Arg Pro Arg Pro Arg Cys Tyr Ser Gly Leu Cys Tyr Ser His Ile
               20                  25                  30
   Ser Gly Leu Asn Gly Leu Gly Leu Ala Ser Pro Pro His Glu Ala Arg
           35                  40                  45
   Gly Val Ala Leu Thr His Arg Cys Tyr Ser Leu Tyr Ser Ala Ser Pro
       50                  55                  60
   Ile Leu Glu Gly Leu Asn Ala Arg Gly Ile Leu Glu Pro Arg Ser Glu
   65                  70                  75                  80
   Arg Leu Glu Pro Arg Pro Arg Ser Glu Arg Thr His Arg Gly Leu Asn
                   85                  90                  95
   Thr His Arg Leu Glu Leu Tyr Ser Leu Glu Ile Leu Glu Gly Leu Thr
               100                 105                 110
   His Arg His Ile Ser Leu Glu Ala Arg Gly Thr His Arg Ile Leu Glu
           115                 120                 125
   Pro Arg Ser Glu Arg His Ile Ser Ala Leu Ala Pro His Glu Ser Glu
       130                 135                 140
   Arg Ala Ser Asn Leu Glu Pro Arg Ala Ser Asn Ile Leu Glu Ser Glu
   145                 150                 155                 160
   Arg Ala Arg Gly Ile Leu Glu Thr Tyr Arg Val Ala Leu Ser Glu Arg
                   165                 170                 175
   Ile Leu Glu Ala Ser Pro Val Ala Leu Thr His Arg Leu Glu Gly Leu
               180                 185                 190
   Asn Gly Leu Asn Leu Glu Gly Leu Ser Glu Arg His Ile Ser Ser Glu
           195                 200                 205
   Arg Pro His Glu Thr Tyr Arg Ala Ser Asn Leu Glu Ser Glu Arg Leu
       210                 215                 220
   Tyr Ser Val Ala Leu Thr His Arg His Ile Ser Ile Leu Glu Gly Leu
   225                 230                 235                 240
   Ile Leu Glu Ala Arg Gly Ala Ser Asn Thr His Arg Ala Arg Gly Ala
                   245                 250                 255
   Ser Asn Leu Glu Thr His Arg Thr Tyr Arg Ile Leu Glu Ala Ser Pro
               260                 265                 270
   Pro Arg Ala Ser Pro Ala Leu Ala Leu Glu Leu Tyr Ser Gly Leu Leu
           275                 280                 285
   Glu Pro Arg Leu Glu Leu Glu Leu Tyr Ser Pro His Glu Leu Glu Gly
       290                 295                 300
   Leu Tyr Ile Leu Glu Pro His Glu Ala Ser Asn Thr His Arg Gly Leu
   305                 310                 315                 320
   Tyr Leu Glu Leu Tyr Ser Met Glu Thr Pro His Glu Pro Arg Ala Ser
                   325                 330                 335
   Pro Leu Glu Thr His Arg Leu Tyr Ser Val Ala Leu Thr Tyr Arg Ser
               340                 345                 350
   Glu Arg Thr His Arg Ala Ser Pro Ile Leu Glu Pro His Glu Pro His
           355                 360                 365
   Glu Ile Leu Glu Leu Glu Gly Leu Ile Leu Glu Thr His Arg Ala Ser
       370                 375                 380
   Pro Ala Ser Asn Pro Arg Thr Tyr Arg Met Glu Thr Thr His Arg Ser
   385                 390                 395                 400
   Glu Arg Ile Leu Glu Pro Arg Val Ala Leu Ala Ser Asn Ala Leu Ala
                   405                 410                 415
   Pro His Glu Gly Leu Asn Gly Leu Tyr Leu Glu Cys Tyr Ser Ala Ser
               420                 425                 430
   Asn Gly Leu Thr His Arg Leu Glu Thr His Arg Leu Glu Leu Tyr Ser
           435                 440                 445
   Leu Glu Thr Tyr Arg Ala Ser Asn Ala Ser Asn Gly Leu Tyr Pro His
       450                 455                 460
   Glu Thr His Arg Ser Glu Arg Val Ala Leu Gly Leu Asn Gly Leu Tyr
   465                 470                 475                 480
   Thr Tyr Arg Ala Leu Ala Pro His Glu Ala Ser Asn Gly Leu Tyr Thr
                   485                 490                 495
   His Arg Leu Tyr Ser Leu Glu Ala Ser Pro Ala Leu Ala Val Ala Leu
               500                 505                 510
   Thr Tyr Arg Leu Glu Ala Ser Asn Leu Tyr Ser Ala Ser Asn Leu Tyr
           515                 520                 525
   Ser Thr Tyr Arg Leu Glu Thr His Arg Val Ala Leu Ile Leu Glu Ala
       530                 535                 540
   Ser Pro Leu Tyr Ser Ala Ser Pro Ala Leu Ala Pro His Glu Gly Leu
   545                 550                 555                 560
   Tyr Gly Leu Tyr Val Ala Leu Thr Tyr Arg Ser Glu Arg Gly Leu Tyr
                   565                 570                 575
   Pro Arg Ser Glu Arg Leu Glu Leu Glu Ala Ser Pro Val Ala Leu Ser
               580                 585                 590
   Glu Arg Gly Leu Asn Thr His Arg Ser Glu Arg Val Ala Leu Thr His
           595                 600                 605
   Arg Ala Leu Ala Leu Glu Pro Arg Ser Glu Arg Leu Tyr Ser Gly Leu
       610                 615                 620
   Tyr Leu Glu Gly Leu His Ile Ser Leu Glu Leu Tyr Ser Gly Leu Leu
   625                 630                 635                 640
   Glu Ile Leu Glu Ala Leu Ala Ala Arg Gly Ala Ser Asn Thr His Arg
                   645                 650                 655
   Thr Arg Pro Thr His Arg Leu Glu
               660
<210> 4
   <211> 857
   <212> PRT
   <213> Artificial Sequence
<220>
   <223> Sequenz 4
<400> 4
   Gly Leu Tyr Met Glu Thr Gly Leu Tyr Cys Tyr Ser Ser Glu Arg Ser
   1               5                   10                  15
   Glu Arg Pro Arg Pro Arg Cys Tyr Ser Gly Leu Cys Tyr Ser His Ile
               20                  25                  30
   Ser Gly Leu Asn Gly Leu Gly Leu Ala Ser Pro Pro His Glu Ala Arg
           35                  40                  45
   Gly Val Ala Leu Thr His Arg Cys Tyr Ser Leu Tyr Ser Ala Ser Pro
       50                  55                  60
   Ile Leu Glu Gly Leu Asn Ala Arg Gly Ile Leu Glu Pro Arg Ser Glu
   65                  70                  75                  80
   Arg Leu Glu Pro Arg Pro Arg Ser Glu Arg Thr His Arg Gly Leu Asn
                   85                  90                  95
   Thr His Arg Leu Glu Leu Tyr Ser Leu Glu Ile Leu Glu Gly Leu Thr
               100                 105                 110
   His Arg His Ile Ser Leu Glu Ala Arg Gly Thr His Arg Ile Leu Glu
           115                 120                 125
   Pro Arg Ser Glu Arg His Ile Ser Ala Leu Ala Pro His Glu Ser Glu
       130                 135                 140
   Arg Ala Ser Asn Leu Glu Pro Arg Ala Ser Asn Ile Leu Glu Ser Glu
   145                 150                 155                 160
   Arg Ala Arg Gly Ile Leu Glu Thr Tyr Arg Val Ala Leu Ser Glu Arg
                   165                 170                 175
   Ile Leu Glu Ala Ser Pro Val Ala Leu Thr His Arg Leu Glu Gly Leu
               180                 185                 190
   Asn Gly Leu Asn Leu Glu Gly Leu Ser Glu Arg His Ile Ser Ser Glu
           195                 200                 205
   Arg Pro His Glu Thr Tyr Arg Ala Ser Asn Leu Glu Ser Glu Arg Leu
       210                 215                 220
   Tyr Ser Val Ala Leu Thr His Arg His Ile Ser Ile Leu Glu Gly Leu
   225                 230                 235                 240
   Ile Leu Glu Ala Arg Gly Ala Ser Asn Thr His Arg Ala Arg Gly Ala
                   245                 250                 255
   Ser Asn Leu Glu Thr His Arg Thr Tyr Arg Ile Leu Glu Ala Ser Pro
               260                 265                 270
   Pro Arg Ala Ser Pro Ala Leu Ala Leu Glu Leu Tyr Ser Gly Leu Leu
           275                 280                 285
   Glu Pro Arg Leu Glu Leu Glu Leu Tyr Ser Pro His Glu Leu Glu Gly
       290                 295                 300
   Leu Tyr Ile Leu Glu Pro His Glu Ala Ser Asn Thr His Arg Gly Leu
   305                 310                 315                 320
   Tyr Leu Glu Leu Tyr Ser Met Glu Thr Pro His Glu Pro Arg Ala Ser
                   325                 330                 335
   Pro Leu Glu Thr His Arg Leu Tyr Ser Val Ala Leu Thr Tyr Arg Ser
               340                 345                 350
   Glu Arg Thr His Arg Ala Ser Pro Ile Leu Glu Pro His Glu Pro His
           355                 360                 365
   Glu Ile Leu Glu Leu Glu Gly Leu Ile Leu Glu Thr His Arg Ala Ser
       370                 375                 380
   Pro Ala Ser Asn Pro Arg Thr Tyr Arg Met Glu Thr Thr His Arg Ser
   385                 390                 395                 400
   Glu Arg Ile Leu Glu Pro Arg Val Ala Leu Ala Ser Asn Ala Leu Ala
                   405                 410                 415
   Pro His Glu Gly Leu Asn Gly Leu Tyr Leu Glu Cys Tyr Ser Ala Ser
               420                 425                 430
   Asn Gly Leu Thr His Arg Leu Glu Thr His Arg Leu Glu Leu Tyr Ser
           435                 440                 445
   Leu Glu Thr Tyr Arg Ala Ser Asn Ala Ser Asn Gly Leu Tyr Pro His
       450                 455                 460
   Glu Thr His Arg Ser Glu Arg Val Ala Leu Gly Leu Asn Gly Leu Tyr
   465                 470                 475                 480
   Thr Tyr Arg Ala Leu Ala Pro His Glu Ala Ser Asn Gly Leu Tyr Thr
                   485                 490                 495
   His Arg Leu Tyr Ser Leu Glu Ala Ser Pro Ala Leu Ala Val Ala Leu
               500                 505                 510
   Thr Tyr Arg Leu Glu Ala Ser Asn Leu Tyr Ser Ala Ser Asn Leu Tyr
           515                 520                 525
   Ser Thr Tyr Arg Leu Glu Thr His Arg Val Ala Leu Ile Leu Glu Ala
       530                 535                 540
   Ser Pro Leu Tyr Ser Ala Ser Pro Ala Leu Ala Pro His Glu Gly Leu
   545                 550                 555                 560
   Tyr Gly Leu Tyr Val Ala Leu Thr Tyr Arg Ser Glu Arg Gly Leu Tyr
                   565                 570                 575
   Pro Arg Ser Glu Arg Leu Glu Leu Glu Ala Ser Pro Val Ala Leu Ser
               580                 585                 590
   Glu Arg Gly Leu Asn Thr His Arg Ser Glu Arg Val Ala Leu Thr His
           595                 600                 605
   Arg Ala Leu Ala Leu Glu Pro Arg Ser Glu Arg Leu Tyr Ser Gly Leu
       610                 615                 620
   Tyr Leu Glu Gly Leu His Ile Ser Leu Glu Leu Tyr Ser Gly Leu Leu
   625                 630                 635                 640
   Glu Ile Leu Glu Ala Leu Ala Ala Arg Gly Ala Ser Asn Thr His Arg
                   645                 650                 655
   Thr Arg Pro Thr His Arg Leu Glu Leu Tyr Ser Leu Tyr Ser Thr His
               660                 665                 670
   Arg Leu Glu Pro Arg Ser Glu Arg Leu Tyr Ser Gly Leu Leu Tyr Ser
           675                 680                 685
   Pro His Glu Thr His Arg Ser Glu Arg Leu Glu Leu Glu Val Ala Leu
       690                 695                 700
   Ala Leu Ala Thr His Arg Leu Glu Thr His Arg Thr Tyr Arg Pro Arg
   705                 710                 715                 720
   Ser Glu Arg His Ile Ser Cys Tyr Ser Cys Tyr Ser Ala Leu Ala Pro
                   725                 730                 735
   His Glu Ala Arg Gly Ala Ser Asn Leu Glu Pro Arg Leu Tyr Ser Leu
               740                 745                 750
   Tyr Ser Gly Leu Gly Leu Asn Ala Ser Asn Pro His Glu Ser Glu Arg
           755                 760                 765
   Pro His Glu Ser Glu Arg Ile Leu Glu Pro His Glu Gly Leu Ala Ser
       770                 775                 780
   Asn Pro His Glu Ser Glu Arg Leu Tyr Ser Gly Leu Asn Cys Tyr Ser
   785                 790                 795                 800
   Gly Leu Ser Glu Arg Thr His Arg Val Ala Leu Ala Arg Gly Leu Tyr
                   805                 810                 815
   Ser Ala Leu Ala Ala Ser Pro Ala Ser Asn Gly Leu Thr His Arg Leu
               820                 825                 830
   Glu Thr Tyr Arg Ser Glu Arg Ala Leu Ala Ile Leu Glu Pro His Glu
           835                 840                 845
   Gly Leu Gly Leu Ala Ser Asn Gly Leu
       850                 855
<210> 5
   <211> 2012
   <212> DNA
   <213> Artificial Sequence
<220>
   <221> source
   <222> 1..2012
   <223> /mol_type="unassigned DNA" /note="Sequenz" /organism="Artificial Sequence"
<400> 5
   gatatcctag cttttctctt ctgtcaaccc cacacgcctt tggcacagat atcacaatga 60
   agtgggtaac ctttatttcc cttctttttc tctttagctc ggcttattcc aggggtgtgt 120
   ttcgtcgaga tgcacacaag agtgaggttg ctcatcggtt taaagatttg ggagaagaaa 180
   atttcaaagc cttggtgttg attgcctttg ctcagtatct tcagcagtgt ccatttgaag 240
   atcatgtaaa attagtgaat gaagtaactg aatttgcaaa aacatgtgtt gctgatgagt 300
   cagctgaaaa ttgtgacaaa tcacttcata ccctttttgg agacaaatta tgcacagttg 360
   caactcttcg tgaaacctat ggtgaaatgg ctgactgctg tgcaaaacaa gaacctgaga 420
   gaaatgaatg cttcttgcaa cacaaagatg acaacccaaa cctcccccga ttggtgagac 480
   cagaggttga tgtgatgtgc actgcttttc atgacaatga agagacattt ttgaaaaaat 540
   acttatatga aattgccaga agacatcctt acttttatgc cccggaactc cttttctttg 600
   ctaaaaggta taaagctgct tttacagaat gttgccaagc tgctgataaa gctgcctgcc 660
   tgttgccaaa gctcatgagg ccggcggact tgctgcagct ggtgctgctg ctcgacctgc 720
   ccagggacct gggcggaatg gggtgttcgt ctccaccctg cgagtgccat caggaggagg 780
   acttcagagt cacctgcaag gatattcaac gcatccccag cttaccgccc agtacgcaga 840
   ctctgaagct tattgagact cacctgagaa ctattccaag tcatgcattt tctaatctgc 900
   ccaatatttc cagaatctac gtatctatag atgtgactct gcagcagctg gaatcacact 960
   ccttctacaa tttgagtaaa gtgactcaca tagaaattcg gaataccagg aacttaactt 1020
   acatagaccc tgatgccctc aaagagctcc ccctcctaaa gttccttggc attttcaaca 1080
   ctggacttaa aatgttccct gacctgacca aagtttattc cactgatata ttctttatac 1140
   ttgaaattac agacaaccct tacatgacgt caatccctgt gaatgctttt cagggactat 1200
   gcaatgaaac cttgacactg aagctgtaca acaatggctt tacttcagtc caaggatatg 1260
   ctttcaatgg gacaaagctg gatgctgttt acctaaacaa gaataaatac ctgacagtta 1320
   ttgacaaaga tgcatttgga ggagtataca gtggaccaag cttgctggac gtgtctcaaa 1380
   ccagtgtcac tgcccttcca tccaaaggcc tggagcacct gaaggaactg atagcaagaa 1440
   acacctggac tcttaagaaa cttccacttt ccttgagttt ccttcacctc acacgggctg 1500
   acctttctta cccaagccac tgctgtgctt ttaagaatca gaagaaaatc agaggaatcc    1560
   ttgagtcctt gatgtgtaat gagagcagta tgcagagctt gcgccagaga aaatctgtga    1620
   atgccttgaa tagccccctc caccaggaat atgaagagaa tctgggtgac agcattgttg    1680
   ggtacaagga aaagtccaag ttccaggata ctcataacaa cgctcattat tacgtcttct    1740
   ttgaagaaca agaggatgag atcattggtt ttggccagga gctcaaaaac ccccaggaag    1800
   agactctaca agcttttgac agccattatg actacaccat atgtggggac agtgaagaca    1860
   tggtgtgtac ccccaagtcc gatgagttca acccgtgtga agacataatg ggctacaagt    1920
   tcctgagaat ctgtaaacgc caggctcagg cataccgggg gcagagggtt cctccaaaga    1980
   acagcactga tattcaggtt caaaaggtta cc                                  2012
<210> 6
   <211> 1802
   <212> PRT
   <213> Artificial Sequence
<220>
   <223> Sequenz 6
<400> 6
   Met Glu Thr Leu Tyr Ser Thr Arg Pro Val Ala Leu Thr His Arg Pro
   1               5                   10                  15
   His Glu Ile Leu Glu Ser Glu Arg Leu Glu Leu Glu Pro His Glu Leu
               20                  25                  30
   Glu Pro His Glu Ser Glu Arg Ser Glu Arg Ala Leu Ala Thr Tyr Arg
           35                  40                  45
   Ser Glu Arg Ala Arg Gly Gly Leu Tyr Val Ala Leu Pro His Glu Ala
       50                  55                  60
   Arg Gly Ala Arg Gly Ala Ser Pro Ala Leu Ala His Ile Ser Leu Tyr
   65                  70                  75                  80
   Ser Ser Glu Arg Gly Leu Val Ala Leu Ala Leu Ala His Ile Ser Ala
                   85                  90                  95
   Arg Gly Pro His Glu Leu Tyr Ser Ala Ser Pro Leu Glu Gly Leu Tyr
               100                 105                 110
   Gly Leu Gly Leu Ala Ser Asn Pro His Glu Leu Tyr Ser Ala Leu Ala
           115                 120                 125
   Leu Glu Val Ala Leu Leu Glu Ile Leu Glu Ala Leu Ala Pro His Glu
       130                 135                 140
   Ala Leu Ala Gly Leu Asn Thr Tyr Arg Leu Glu Gly Leu Asn Gly Leu
   145                 150                 155                 160
   Asn Cys Tyr Ser Pro Arg Pro His Glu Gly Leu Ala Ser Pro His Ile
                   165                 170                 175
   Ser Val Ala Leu Leu Tyr Ser Leu Glu Val Ala Leu Ala Ser Asn Gly
               180                 185                 190
   Leu Val Ala Leu Thr His Arg Gly Leu Pro His Glu Ala Leu Ala Leu
           195                 200                 205
   Tyr Ser Thr His Arg Cys Tyr Ser Val Ala Leu Ala Leu Ala Ala Ser
       210                 215                 220
   Pro Gly Leu Ser Glu Arg Ala Leu Ala Gly Leu Ala Ser Asn Cys Tyr
   225                 230                 235                 240
   Ser Ala Ser Pro Leu Tyr Ser Ser Glu Arg Leu Glu His Ile Ser Thr
                   245                 250                 255
   His Arg Leu Glu Pro His Glu Gly Leu Tyr Ala Ser Pro Leu Tyr Ser
               260                 265                 270
   Leu Glu Cys Tyr Ser Thr His Arg Val Ala Leu Ala Leu Ala Thr His
               275                 280                 285
   Arg Leu Glu Ala Arg Gly Gly Leu Thr His Arg Thr Tyr Arg Gly Leu
           290                 295                 300
   Tyr Gly Leu Met Glu Thr Ala Leu Ala Ala Ser Pro Cys Tyr Ser Cys
   305                 310                 315                 320
   Tyr Ser Ala Leu Ala Leu Tyr Ser Gly Leu Asn Gly Leu Pro Arg Gly
                   325                 330                 335
   Leu Ala Arg Gly Ala Ser Asn Gly Leu Cys Tyr Ser Pro His Glu Leu
               340                 345                 350
   Glu Gly Leu Asn His Ile Ser Leu Tyr Ser Ala Ser Pro Ala Ser Pro
           355                 360                 365
   Ala Ser Asn Pro Arg Ala Ser Asn Leu Glu Pro Arg Ala Arg Gly Leu
       370                 375                 380
   Glu Val Ala Leu Ala Arg Gly Pro Arg Gly Leu Val Ala Leu Ala Ser
   385                 390                 395                 400
   Pro Val Ala Leu Met Glu Thr Cys Tyr Ser Thr His Arg Ala Leu Ala
                   405                 410                 415
   Pro His Glu His Ile Ser Ala Ser Pro Ala Ser Asn Gly Leu Gly Leu
               420                 425                 430
   Thr His Arg Pro His Glu Leu Glu Leu Tyr Ser Leu Tyr Ser Thr Tyr
           435                 440                 445
   Arg Leu Glu Thr Tyr Arg Gly Leu Ile Leu Glu Ala Leu Ala Ala Arg
       450                 455                 460
   Gly Ala Arg Gly His Ile Ser Pro Arg Thr Tyr Arg Pro His Glu Thr
   465                 470                 475                 480
   Tyr Arg Ala Leu Ala Pro Arg Gly Leu Leu Glu Leu Glu Pro His Glu
                   485                 490                 495
   Pro His Glu Ala Leu Ala Leu Tyr Ser Ala Arg Gly Thr Tyr Arg Leu
               500                 505                 510
   Tyr Ser Ala Leu Ala Ala Leu Ala Pro His Glu Thr His Arg Gly Leu
           515                 520                 525
   Cys Tyr Ser Cys Tyr Ser Gly Leu Asn Ala Leu Ala Ala Leu Ala Ala
       530                 535                 540
   Ser Pro Leu Tyr Ser Ala Leu Ala Ala Leu Ala Cys Tyr Ser Leu Glu
   545                 550                 555                 560
   Leu Glu Pro Arg Leu Tyr Ser Leu Glu Met Glu Thr Ala Arg Gly Pro
                   565                 570                 575
   Arg Ala Leu Ala Ala Ser Pro Leu Glu Leu Glu Gly Leu Asn Leu Glu
               580                 585                 590
   Val Ala Leu Leu Glu Leu Glu Leu Glu Ala Ser Pro Leu Glu Pro Arg
           595                 600                 605
   Ala Arg Gly Ala Ser Pro Leu Glu Gly Leu Tyr Gly Leu Tyr Met Glu
       610                 615                 620
   Thr Gly Leu Tyr Cys Tyr Ser Ser Glu Arg Ser Glu Arg Pro Arg Pro
   625                 630                 635                 640
   Arg Cys Tyr Ser Gly Leu Cys Tyr Ser His Ile Ser Gly Leu Asn Gly
                   645                 650                 655
   Leu Gly Leu Ala Ser Pro Pro His Glu Ala Arg Gly Val Ala Leu Thr
               660                 665                 670
   His Arg Cys Tyr Ser Leu Tyr Ser Ala Ser Pro Ile Leu Glu Gly Leu
           675                 680                 685
   Asn Ala Arg Gly Ile Leu Glu Pro Arg Ser Glu Arg Leu Glu Pro Arg
       690                 695                 700
   Pro Arg Ser Glu Arg Thr His Arg Gly Leu Asn Thr His Arg Leu Glu
   705                 710                 715                 720
   Leu Tyr Ser Leu Glu Ile Leu Glu Gly Leu Thr His Arg His Ile Ser
                   725                 730                 735
   Leu Glu Ala Arg Gly Thr His Arg Ile Leu Glu Pro Arg Ser Glu Arg
               740                 745                 750
   His Ile Ser Ala Leu Ala Pro His Glu Ser Glu Arg Ala Ser Asn Leu
           755                 760                 765
   Glu Pro Arg Ala Ser Asn Ile Leu Glu Ser Glu Arg Ala Arg Gly Ile
       770                 775                 780
   Leu Glu Thr Tyr Arg Val Ala Leu Ser Glu Arg Ile Leu Glu Ala Ser
   785                 790                 795                 800
   Pro Val Ala Leu Thr His Arg Leu Glu Gly Leu Asn Gly Leu Asn Leu
                   805                 810                 815
   Glu Gly Leu Ser Glu Arg His Ile Ser Ser Glu Arg Pro His Glu Thr
               820                 825                 830
   Tyr Arg Ala Ser Asn Leu Glu Ser Glu Arg Leu Tyr Ser Val Ala Leu
           835                 840                 845
   Thr His Arg His Ile Ser Ile Leu Glu Gly Leu Ile Leu Glu Ala Arg
       850                 855                 860
   Gly Ala Ser Asn Thr His Arg Ala Arg Gly Ala Ser Asn Leu Glu Thr
   865                 870                 875                 880
   His Arg Thr Tyr Arg Ile Leu Glu Ala Ser Pro Pro Arg Ala Ser Pro
                   885                 890                 895
   Ala Leu Ala Leu Glu Leu Tyr Ser Gly Leu Leu Glu Pro Arg Leu Glu
               900                 905                 910
   Leu Glu Leu Tyr Ser Pro His Glu Leu Glu Gly Leu Tyr Ile Leu Glu
           915                 920                 925
   Pro His Glu Ala Ser Asn Thr His Arg Gly Leu Tyr Leu Glu Leu Tyr
       930                 935                 940
   Ser Met Glu Thr Pro His Glu Pro Arg Ala Ser Pro Leu Glu Thr His
   945                 950                 955                 960
   Arg Leu Tyr Ser Val Ala Leu Thr Tyr Arg Ser Glu Arg Thr His Arg
                   965                 970                 975
   Ala Ser Pro Ile Leu Glu Pro His Glu Pro His Glu Ile Leu Glu Leu
               980                 985                 990
   Glu Gly Leu Ile Leu Glu Thr His Arg Ala Ser Pro Ala Ser Asn Pro
           995                 1000                1005
   Arg Thr Tyr Arg Met Glu Thr Thr His Arg Ser Glu Arg Ile Leu Glu
       1010                1015                1020
   Pro Arg Val Ala Leu Ala Ser Asn Ala Leu Ala Pro His Glu Gly Leu
   1025                1030                1035                1040
   Asn Gly Leu Tyr Leu Glu Cys Tyr Ser Ala Ser Asn Gly Leu Thr His
                   1045                1050                1055
   Arg Leu Glu Thr His Arg Leu Glu Leu Tyr Ser Leu Glu Thr Tyr Arg
               1060                1065                1070
   Ala Ser Asn Ala Ser Asn Gly Leu Tyr Pro His Glu Thr His Arg Ser
           1075                1080                1085
   Glu Arg Val Ala Leu Gly Leu Asn Gly Leu Tyr Thr Tyr Arg Ala Leu
       1090                1095                1100
   Ala Pro His Glu Ala Ser Asn Gly Leu Tyr Thr His Arg Leu Tyr Ser
   1105                1110                1115                1120
   Leu Glu Ala Ser Pro Ala Leu Ala Val Ala Leu Thr Tyr Arg Leu Glu
                   1125                1130                1135
   Ala Ser Asn Leu Tyr Ser Ala Ser Asn Leu Tyr Ser Thr Tyr Arg Leu
               1140                1145                1150
   Glu Thr His Arg Val Ala Leu Ile Leu Glu Ala Ser Pro Leu Tyr Ser
           1155                1160                1165
   Ala Ser Pro Ala Leu Ala Pro His Glu Gly Leu Tyr Gly Leu Tyr Val
       1170                1175                1180
   Ala Leu Thr Tyr Arg Ser Glu Arg Gly Leu Tyr Pro Arg Ser Glu Arg
   1185                1190                1195                1200
   Leu Glu Leu Glu Ala Ser Pro Val Ala Leu Ser Glu Arg Gly Leu Asn
                   1205                1210                1215
   Thr His Arg Ser Glu Arg Val Ala Leu Thr His Arg Ala Leu Ala Leu
               1220                1225                1230
   Glu Pro Arg Ser Glu Arg Leu Tyr Ser Gly Leu Tyr Leu Glu Gly Leu
           1235                1240                1245
   His Ile Ser Leu Glu Leu Tyr Ser Gly Leu Leu Glu Ile Leu Glu Ala
       1250                1255                1260
   Leu Ala Ala Arg Gly Ala Ser Asn Thr His Arg Thr Arg Pro Thr His
   1265                1270                1275                1280
   Arg Leu Glu Leu Tyr Ser Leu Tyr Ser Leu Glu Pro Arg Leu Glu Ser
                   1285                1290                1295
   Glu Arg Leu Glu Ser Glu Arg Pro His Glu Leu Glu His Ile Ser Leu
               1300                1305                1310
   Glu Thr His Arg Ala Arg Gly Ala Leu Ala Ala Ser Pro Leu Glu Ser
           1315                1320                1325
   Glu Arg Thr Tyr Arg Pro Arg Ser Glu Arg His Ile Ser Cys Tyr Ser
       1330                1335                1340
   Cys Tyr Ser Ala Leu Ala Pro His Glu Leu Tyr Ser Ala Ser Asn Gly
   1345                1350                1355                1360
   Leu Asn Leu Tyr Ser Leu Tyr Ser Ile Leu Glu Ala Arg Gly Gly Leu
                   1365                1370                1375
   Tyr Ile Leu Glu Leu Glu Gly Leu Ser Glu Arg Leu Glu Met Glu Thr
               1380                1385                1390
   Cys Tyr Ser Ala Ser Asn Gly Leu Ser Glu Arg Ser Glu Arg Met Glu
           1395                1400                1405
   Thr Gly Leu Asn Ser Glu Arg Leu Glu Ala Arg Gly Gly Leu Asn Ala
       1410                1415                1420
   Arg Gly Leu Tyr Ser Ser Glu Arg Val Ala Leu Ala Ser Asn Ala Leu
   1425                1430                1435                1440
   Ala Leu Glu Ala Ser Asn Ser Glu Arg Pro Arg Leu Glu His Ile Ser
                   1445                1450                1455
   Gly Leu Asn Gly Leu Thr Tyr Arg Gly Leu Gly Leu Ala Ser Asn Leu
               1460                1465                1470
   Glu Gly Leu Tyr Ala Ser Pro Ser Glu Arg Ile Leu Glu Val Ala Leu
           1475                1480                1485
   Gly Leu Tyr Thr Tyr Arg Leu Tyr Ser Gly Leu Leu Tyr Ser Ser Glu
       1490                1495                1500
   Arg Leu Tyr Ser Pro His Glu Gly Leu Asn Ala Ser Pro Thr His Arg
   1505                1510                1515                1520
   His Ile Ser Ala Ser Asn Ala Ser Asn Ala Leu Ala His Ile Ser Thr
                   1525                1530                1535
   Tyr Arg Thr Tyr Arg Val Ala Leu Pro His Glu Pro His Glu Gly Leu
               1540                1545                1550
   Gly Leu Gly Leu Asn Gly Leu Ala Ser Pro Gly Leu Ile Leu Glu Ile
           1555                1560                1565
   Leu Glu Gly Leu Tyr Pro His Glu Gly Leu Tyr Gly Leu Asn Gly Leu
       1570                1575                1580
   Leu Glu Leu Tyr Ser Ala Ser Asn Pro Arg Gly Leu Asn Gly Leu Gly
   1585                1590                1595                1600
   Leu Thr His Arg Leu Glu Gly Leu Asn Ala Leu Ala Pro His Glu Ala
                   1605                1610                1615
   Ser Pro Ser Glu Arg His Ile Ser Thr Tyr Arg Ala Ser Pro Thr Tyr
               1620                1625                1630
   Arg Thr His Arg Ile Leu Glu Cys Tyr Ser Gly Leu Tyr Ala Ser Pro
           1635                1640                1645
   Ser Glu Arg Gly Leu Ala Ser Pro Met Glu Thr Val Ala Leu Cys Tyr
       1650                1655                1660
   Ser Thr His Arg Pro Arg Leu Tyr Ser Ser Glu Arg Ala Ser Pro Gly
   1665                1670                1675                1680
   Leu Pro His Glu Ala Ser Asn Pro Arg Cys Tyr Ser Gly Leu Ala Ser
                   1685                1690                1695
   Pro Ile Leu Glu Met Glu Thr Gly Leu Tyr Thr Tyr Arg Leu Tyr Ser
               1700                1705                1710
   Pro His Glu Leu Glu Ala Arg Gly Ile Leu Glu Cys Tyr Ser Leu Tyr
           1715                1720                1725
   Ser Ala Arg Gly Gly Leu Asn Ala Leu Ala Gly Leu Asn Ala Leu Ala
       1730                1735                1740
   Thr Tyr Arg Ala Arg Gly Gly Leu Tyr Gly Leu Asn Ala Arg Gly Val
   1745                1750                1755                1760
   Ala Leu Pro Arg Pro Arg Leu Tyr Ser Ala Ser Asn Ser Glu Arg Thr
                   1765                1770                1775
   His Arg Ala Ser Pro Ile Leu Glu Gly Leu Asn Val Ala Leu Gly Leu
               1780                1785                1790
   Asn Leu Tyr Ser Val Ala Leu Thr His Arg
           1795                1800